# EUROPÄISCHE PATENTSCHRIFT

(11) **EP 4 043 873 B1**
(45) Veröffentlichungstag und Bekanntmachung des Hinweises auf die Patenterteilung: **01.11.2023**
(21) Anmeldenummer: 22155727.5
(22) Anmeldetag: 08.02.2022
(51) Int. Cl.: G01N 33/00, G01N 27/16, E21F 17/18, G08B 21/18

(54) **GASDETEKTIONSVORRICHTUNG UND GASDETEKTIONSVERFAHREN MIT EINEM DETEKTOR UND EINEM MODULATOR**
GAS DETECTION APPARATUS AND GAS DETECTION METHOD USING DETECTOR AND MODULATOR
DISPOSITIF DE DÉTECTION DE GAZ ET PROCÉDÉ DE DÉTECTION DE GAZ AU MOYEN D'UN DÉTECTEUR ET D'UN MODULATEUR

(30) Priorität: 16.02.2021 DE 102021103563
(43) Veröffentlichungstag der Anmeldung: 17.08.2022
(73) Patentinhaber: Dräger Safety AG & Co. KGaA, 23560 Lübeck (DE)
(72) Erfinder: Baesler, Malte, 23558 Lübeck (DE); Balhorn, Sandra, 23558 Lübeck (DE); Osswald, Jürgen, 23558 Lübeck (DE)
(74) Vertreter: Meyer-Gramann, Klaus Dieter

(56) Entgegenhaltungen:
- DE-A1-102005 037 529
- DE-A1-102017 005 713
- US-B2- 10 234 412

## Beschreibung

Die Erfindung betrifft eine Gasdetektionsvorrichtung und ein Gasdetektionsverfahren, welche automatisch einen räumlichen Bereich auf das Vorhandensein mindestens eines Zielgases zu überwachen vermögen. Das oder ein zu detektierendes Zielgas ist bei einem im Betrieb der Gasdetektionsvorrichtung auftretenden Temperaturbereich brennbar. Der zu überwachende Bereich ist beispielsweise ein Bergwerk oder eine Raffinerie oder ein Transportfahrzeug oder eine mit einem brennbaren Fluid betriebene Heizungsanlage oder eine Lagerhalle.

Eine Methode, um ein brennbares Zielgas zu detektieren, umfasst die Schritte, in einer Kammer ein Gasgemisch zu erhitzen und die dabei entstehende Wärmeenergie zu messen. Falls das Gasgemisch ein brennbares Zielgas enthält, so wird das Zielgas oxidiert, und das Oxidieren setzt Wärmeenergie frei. Eine mit der freigesetzten Wärmeenergie korrelierende physikalische Größe wird gemessen. Auch die Erfindung nutzt dieses Prinzip. Ein auf diese Weise arbeitender Sensor wird auch als "Wärmetönungssensor" bezeichnet. Verschiedene solche Sensoren sind bekannt geworden.

Bei dem in EP 0 849 594 A2 beschriebenen Verfahren wird ein katalytischer Sensor gepulst betrieben, wodurch die Temperatur des Sensors zwischen einer minimalen und einer maximalen Temperatur oszilliert. Die minimale Temperatur liegt unterhalb der katalytischen Einsatztemperatur eines Zielgases, die maximale Temperatur oberhalb dieser katalytischen Einsatztemperatur.

Das in DE 10 2005 024 394 B4 beschriebene Verfahren, um eine Gaskonzentration zu messen, verwendet ein thermisches Messelement in Form eines Pellistors. Das Messelement wird gepulst betrieben, wobei zwischen den Pulsen Ruhephasen eingehalten werden, die mindestens halb so lang sind wie die Messphasen. Die Antwort des Messelements auf mindestens einen Puls wird ausgewertet, um die Gaskonzentration in der Umgebung des Messelements zu messen. Hierbei werden elektrische Messgrößen ausgewertet, um transiente Zustände des Messelements zu bestimmen.

Auch in US 10 234 412 B2 wird ein Verfahren beschrieben, bei dem ein katalytischer Sensor gepulst betrieben wird. Eine Stromversorgung des Sensors wird periodisch eingeschaltet und ausgeschaltet. Dadurch verändert sich die Temperatur des Sensors periodisch. Die Dynamik des Sensors beim periodischen Einschalten und Ausschalten wird ausgewertet, um zu erkennen, von welcher Art ein Zielgas ist.

In der Beschreibungseinleitung von DE 10 2017 005 713 A1 wird ein Doppeldetektor beschrieben, der zwei gleichartige Pellistoren 12, 22 in jeweils einem Hohlraum 10 bzw. 20 umfasst. Eine Seite des ersten Hohlraums 10 ist für Gas durchlässig, sodass Gas aus der Umgebung in den ersten Hohlraum 10 gelangen kann. Der zweite Hohlraum 20 steht nur über eine kleine Diffusionsöffnung 28 mit der Umgebung in einer Fluidverbindung, wobei die Diffusionsöffnung 28 sich in einer ansonsten gasdichten Diffusionsbarriere 24 befindet.

In der Figurenbeschreibung von DE 10 2017 005 713 A1 wird ein solcher Doppeldetektor beschrieben. Eine Auswerteeinheit wertet die Signale von den beiden gleichartigen Pellistoren 12, 22 aus und verwendet das Signal des zweiten Pellistors 22 als Referenz-Signal, mit dem das Signal des ersten Pellistors 12 verglichen werden.

Der Wärmetönungssensor von DE 10 2017 011 530 A1 umfasst ein Gehäuse 110 mit einem Gaseinlass 111 und einem Gasauslass 112. Ein Gasstrom mit einem zu untersuchenden Gasgemisch wird vom Gaseinlass 111 zum Gasauslass 112 gefördert. Der Gasstrom umströmt oder durchströmt ein Messelement 130. Das Messelement 130 verbrennt katalytisch einen Teil des Gasstroms und misst die beim Verbrennen freiwerdende Wärmemenge. Ein Sensor 160 ist ähnlich wie das Messelement 130 aufgebaut und wird ebenfalls von dem Gasstrom umströmt, verwendet aber ein katalytisch inaktives Material, während das Messelement 130 ein katalytisch aktives Material aufweist. Das Messsignal des Sensors 160 lässt sich dafür verwenden, um Umweltänderungen rechnerisch zu kompensieren. In einer Ausgestaltung messen mindestens zwei Temperatur-Sensoren 380 und 390 die Temperatur eines katalytisch aktiven Materials 333.

Der Gassensor, der in DE 10 2005 037 529 A1 beschrieben wird, vermag Kohlendioxid zu detektieren und umfasst ein Detektionselement 1, einen Filter 2 und zwei Heizelemente 3 und 4, die in einem Gehäuse 6 mit einer diffusionsbegrenzenden Öffnung 5 angeordnet sind. Das Heizelement 3 befindet sich zwischen dem Filter 2 und dem Detektionselement 1, das Heizelement 4 zwischen der Öffnung 5 und dem Filter 2. Der Filter 2 lässt Kohlendioxid passieren, filtert aber Ethanol und andere störende Gase heraus und absorbiert sie. Der Filter 2 übt die filternde Wirkung nur in einer Messphase aus. In einer Regenerationsphase erhitzten die Heizelemente 3 und 4 den Filter 2, sodass der Filter 2 die zuvor absorbierten störenden Gase wieder abgibt (desorbiert). Bevorzugt umfasst der Gassensor zwei parallele Sensorsysteme, wobei ein Sensorsystem sich in der Messphase und ein Sensorsystem sich in der Regenerationsphase befinden kann.

Der Erfindung liegt die Aufgabe zugrunde, eine Gasdetektionsvorrichtung bereitzustellen,
- wobei die Gasdetektionsvorrichtung eine Detektorkammer und einen Detektor in der Detektorkammer umfasst,
- wobei der Detektor ein zu detektierendes Zielgas zu oxidieren vermag,
- wobei ein Oxidieren des Zielgases in der Detektorkammer die Temperatur des Detektors vergrößert und
- wobei die Gasdetektionsvorrichtung mit höherer Zuverlässigkeit als bekannte Gasdetektionsvorrichtungen das Vorhandensein eines brennbaren Zielgases zu detektieren vermag.

Der Erfindung liegt weiterhin die Aufgabe zugrunde, ein Verfahren bereitzustellen, welches unter Verwendung einer solchen Gasdetektionsvorrichtung mit höherer Zuverlässigkeit das Vorhandensein eines brennbaren Zielgases zu detektieren vermag.

Die Aufgabe wird durch eine Gasdetektionsvorrichtung mit den Merkmalen des Anspruchs 1 und durch ein Gasdetektionsverfahren mit den Merkmalen des Anspruchs 10 gelöst. Vorteilhafte Ausgestaltungen sind in den Unteransprüchen angegeben. Vorteilhafte Ausgestaltungen der erfindungsgemäßen Gasdetektionsvorrichtung sind, soweit sinnvoll, auch vorteilhafte Ausgestaltungen des erfindungsgemäßen Gasdetektionsverfahrens und umgekehrt.

Die erfindungsgemäße Gasdetektionsvorrichtung und das erfindungsgemäße Gasdetektionsverfahren vermögen automatisch einen räumlichen Bereich darauf zu überwachen, ob in diesem Bereich ein brennbares Zielgas vorhanden ist oder nicht. Die Vorrichtung und das Verfahren vermögen in der Regel auch den Fall zu detektieren, dass gleichzeitig mehrere brennbare Zielgase vorhanden sind.

Die erfindungsgemäße Gasdetektionsvorrichtung umfasst
- einen Detektor,
- einen Modulator,
- eine Detektorkammer,
- eine Modulatorkammer,
- mindestens einen Detektor-Sensor und
- eine signalverarbeitende Auswerteeinheit.

Die Modulatorkammer umgibt den Modulator. Die Modulatorkammer steht in einer äußeren Fluidverbindung mit dem zu überwachenden Bereich. Zwischen der Modulatorkammer und der Detektorkammer besteht eine innere Fluidverbindung.

Dank dieser beiden Fluidverbindungen kann ein Gasgemisch aus dem zu überwachenden Bereich durch die Modulatorkammer hindurch in die Detektorkammer fließen.

Der Begriff "Kammer" bezeichnet jeden Gegenstand, der einen Raum - bis auf definierte Öffnungen und unvermeidliche Spalten und Schlitze - fluiddicht umschließt.

Unter einer "Fluidverbindung" wird eine mechanische Verbindung verstanden, durch die ein Fluid, insbesondere ein Gas oder ein Gasgemisch, in die jeweilige Kammer hinein und optional auch wieder aus der Kammer heraus fließen kann.

Die Detektorkammer trennt den Detektor von dem zu überwachenden Bereich. Optional trägt zusätzlich die Modulatorkammer dazu bei, den Detektor von dem zu überwachenden Bereich zu trennen. Die Querschnittsfläche einer Fluidverbindung zwischen der Detektorkammer und dem zu überwachenden Bereich beträgt höchstens 25 % der Querschnittsfläche der inneren Fluidverbindung, bevorzugt höchstens 10 %. In einer besonders bevorzugten Ausgestaltung trennt die Detektorkammer den Detektor gasdicht von dem zu überwachenden Bereich, sodass überhaupt kein Fluid aus dem zu überwachenden Bereich direkt in die Detektorkammer fließen kann, abgesehen von in der Regel unvermeidlichen Spalten. Bei den Prozentangaben und der bevorzugten Eigenschaft, dass die Detektorkammer den Detektor gasdicht von dem Bereich trennt, ist die innere Fluidverbindung nicht berücksichtigt.

Die Gasdetektionsvorrichtung vermag sowohl an den Detektor als auch an den Modulator jeweils eine elektrische Spannung anzulegen. Falls an den Detektor eine elektrische Spannung angelegt wird, so fließt elektrischer Strom durch den Detektor. Als Folge erhitzt sich aufgrund des durch den Detektor fließenden elektrischen Stroms ein Bauteil des Detektors, beispielsweise ein von Strom durchflossener Draht. Falls an den Modulator eine elektrische Spannung angelegt wird, so fließt elektrischer Strom durch den Modulator, und ein Bauteil des Modulators erhitzt sich aufgrund des durch den Modulator fließenden elektrischen Stroms.

Der Detektor und der Modulator sind wie folgt ausgestaltet: Ein Erhitzen des Detektors bewirkt, dass ein zu detektierendes Zielgas, welches sich in der Detektorkammer befindet, oxidiert wird. Dieses Oxidieren hängt von der Temperatur des Detektors ab, wobei durchfließender elektrischer Strom das Bauteil des Detektors erhitzt. Ein Erhitzen des Modulators bewirkt, dass ein zu detektierendes Zielgas, welches sich in der Modulatorkammer befindet, oxidiert wird, wobei durchfließender elektrischer Strom das Bauteil des Modulators erhitzt. Dieses Oxidieren hängt von der Temperatur des Modulators ab.

Der Detektor ist weiterhin wie folgt ausgestaltet: Wenn ein Zielgas in der Detektorkammer oxidiert wird, so wird die Temperatur des Detektors vergrößert.

Jeweils ein Bauteil des Detektors und des Modulators wird von elektrischem Strom durchflossen, wenn elektrische Spannung angelegt wird, und erhitzt sich hierbei. Unter der "Temperatur" des Detektors bzw. Modulators wird eine mittlere Temperatur dieses stromleitenden Bauteils verstanden, wobei die Mittelung auf die räumliche Ausdehnung des stromleitenden Bauteils bezogen ist. Selbstverständlich kann das erhitzte Bauteil weitere Bereiche des Detektors bzw. Modulators erhitzen.

Der oder mindestens ein Detektor-Sensor vermag eine Detektions-Größe des Detektors zu messen. In einer Alternative ist die Detektions-Größe die Detektor-Temperatur, welche durch das Oxidieren des Zielgases vergrößert wird. In einer anderen Alternative ist die Detektions-Größe ein Parameter des Detektors, wobei dieser Parameter mit der Detektor-Temperatur korreliert. Insbesondere ist der Parameter die elektrische Spannung, die am Detektor anliegt, oder die Stärke des elektrischen Stroms, welcher durch den Detektor fließt, oder der elektrische Widerstand des Detektors oder die vom Detektor aufgenommene elektrische Leistung. Bekanntlich verändert sich in vielen Fällen der elektrische Widerstand eines von elektrischem Strom durchflossenen Bauteils, wenn die Temperatur in der Umgebung und damit die Temperatur des Bauteils sich ändert. Diese Abhängigkeit hängt vom Material des stromdurchflossenen Bauteils ab und ist bekannt oder lässt sich empirisch bestimmen. Möglich ist, dass mehrere Detektions-Größen gemessen werden.

Erfindungsgemäß vermag die Gasdetektionsvorrichtung an den Modulator eine elektrische Spannung anzulegen. Die elektrische Spannung wird erfindungsgemäß dergestalt angelegt, dass ein oszillierender elektrischer Strom durch den Modulator fließt und die Temperatur des stromdurchflossenen Modulators daher ebenfalls zeitlich oszilliert. Unter einer "Oszillation" einer physikalischen Größe wird der Vorgang verstanden, dass die physikalische Größe in mehreren aufeinanderfolgenden Zunahme-Zeiträumen zunimmt und in mehreren aufeinanderfolgenden Abnahme-Zeiträumen abnimmt, wobei zeitlich hinter jedem Zunahme-Zeitraum jeweils ein Abnahme-Zeitraum liegt.

Erfindungsgemäß vermag die Gasdetektionsvorrichtung an den Detektor eine elektrische Spannung anzulegen, sodass ein elektrischer Strom durch den Detektor fließt und die Detektor-Temperatur vergrößert wird. In einer Alternative oszilliert die Detektor-Temperatur, die durch das Anlegen der elektrischen Spannung und die resultierende Erhitzung des Detektor-Bauteils bewirkt wird, zeitlich überhaupt nicht. In einer anderen Alternative oszilliert auch die Detektor-Temperatur, aber mit einer geringeren Amplitude als die Modulator-Temperatur.

Die Auswerteeinheit vermag das Vorhandensein eines brennbaren Zielgases automatisch zu detektieren. Für diese Detektion wertet die Auswerteeinheit Messwerte des oder mindestens eines Detektor-Sensors aus und entscheidet automatisch, ob die Detektions-Größe des Detektors synchron mit der Modulator-Temperatur oszilliert oder nicht. Die Auswerteeinheit entscheidet hierbei, ob das synchrone Oszillieren im gesamten Auswerte-Zeitraum oder wenigstens in einem Teil des Auswerte-Zeitraums stattfindet oder nicht. Bei einem synchronen Oszillieren ist ein brennbares Zielgas vorhanden und detektiert. Bevorzugt vernachlässigt die Auswerteeinheit bei der Entscheidung ein unvermeidbares Rauschen im zeitlichen Verlauf der Detektions-Größe und berücksichtigt bevorzugt nur ein Oszillieren mit einer Amplitude oberhalb einer vorgegebenen minimalen Amplitude. Bevorzugt entscheidet die Auswerteeinheit automatisch, dass kein brennbares Zielgas vorhanden ist, wenn im Auswerte-Zeitraum kein synchrones zeitliches Oszillieren - oder wenigstens kein Oszillieren oberhalb der minimalen Amplitude - detektiert ist.

Ein Gasgemisch, welches das zu detektierende Zielgas oder ein zu detektierendes Zielgas enthalten kann, fließt aus dem zu überwachenden Bereich durch die äußere Fluidverbindung hindurch in die Modulatorkammer. Dieses Gasgemisch umgibt den Modulator. Ein Teil dieses Gasgemisches fließt durch die innere Fluidverbindung hindurch in die Detektorkammer.

Erfindungsgemäß wird an den Modulator eine elektrische Spannung angelegt. Durch das Anlegen der Spannung fließt ein elektrischer Strom durch den Modulator, und dadurch wird ein Bauteil des Modulators erhitzt. Die elektrische Spannung wird dergestalt angelegt, dass die aus dem Erhitzen resultierende Temperatur des Modulators über die Zeit oszilliert.

Falls das Gasgemisch in dem zu überwachenden Bereich mindestens ein brennbares Zielgas enthält, so fließt dieses Zielgas mitsamt dem Gasgemisch durch die äußere Fluidverbindung in die Modulatorkammer. Ein in der Modulatorkammer befindliches Zielgas wird oxidiert, und zwar abhängig von der oszillierenden Temperatur des Modulators und damit abhängig von der Erhitzung des Modulators, wobei die angelegte elektrische Spannung diese Erhitzung bewirkt hat. Natürlich ist es möglich, dass in dem zu überwachenden Bereich und damit auch in der Modulatorkammer kein brennbares Zielgas vorhanden ist und der erhitzte Modulator daher kein Zielgas zu oxidieren vermag.

Dann, wenn die Modulator-Temperatur einen hohen Wert annimmt, wird ein großer Teil oder sogar alles brennbare Zielgas in der Modulatorkammer oxidiert. Wenn die Modulator-Temperatur einen niedrigen Wert annimmt, so wird nur ein geringer Teil oder sogar überhaupt kein Zielgas in der Modulatorkammer oxidiert. In vielen Fällen wird in der Regel ein umso höherer Anteil des brennbaren Zielgases in der Modulatorkammer oxidiert, je höher die Temperatur des Modulators ist. Natürlich vermag der erhitzte Modulator nur dann Zielgas zu oxidieren, wenn in dem zu überwachenden Bereich und damit auch in der Modulatorkammer ein brennbares Zielgas vorhanden ist.

Der Begriff "oxidieren" umfasst einen Vorgang, bei welchem das brennbare Zielgas chemisch oxidiert wird, aber nicht notwendigerweise mit einer offenen Flamme verbrennt oder gar durch einen unkontrollierten Oxidationsvorgang explodiert. Der Begriff "oxidieren" umfasst auch ein Verbrennen mit einer offenen Flamme oder sogar eine Explosion, welche aufgrund einer ausreichend hohen Temperatur des Modulators oder des Detektors auftreten kann, wenn das brennbare Zielgas eine ausreichend hohe Konzentration in der jeweiligen Kammer erreicht.

Das Gasgemisch gelangt aus dem zu überwachenden Bereich durch die äußere Fluidverbindung hindurch in die Modulatorkammer und durch die innere Fluidverbindung hindurch in die Detektorkammer. Weil am Detektor eine elektrische Spannung angelegt ist, wird das Detektor-Bauteil erhitzt. Der Detektor oxidiert brennbares Zielgas, welches in der Detektorkammer vorhanden ist - aber natürlich nur, wenn das Gasgemisch in der Detektorkammer ein brennbares Zielgas mit ausreichend großer Konzentration aufweist. Der Vorgang, dass brennbares Zielgas in der Detektorkammer oxidiert wird, setzt Wärmeenergie frei, führt dem Detektor wenigstens einen Teil der freigesetzten Wärmeenergie zu und erhöht daher die Temperatur des Detektors - verglichen mit einem Zustand ohne brennbaren Zielgas in der Detektorkammer. Die bewirkte Temperaturerhöhung - im Vergleich zur Temperatur, die ausschließlich durch die angelegte elektrische Spannung hervorgerufen wird - korreliert mit der Menge und / oder der Konzentration des Zielgases in der Detektorkammer. Eine große Menge oder eine große Konzentration des Zielgases führt zu einem hohen Temperaturanstieg, eine niedrige Menge oder Konzentration zu einem niedrigen Temperaturanstieg.

Erfindungsgemäß trennt die Detektorkammer den Detektor im Wesentlichen von der Umgebung ab - nämlich bis auf die innere Fluidverbindung zur Modulatorkammer, bis auf die optionalen Öffnungen, deren Querschnittsfläche höchstens 25 % der Querschnittsfläche der inneren Fluidverbindung beträgt, und bis auf unvermeidliche Schlitze oder Spalten, die in der Regel insbesondere aufgrund von Fertigungstoleranzen und Materialveränderungen im Laufe des Einsatzes auftreten. Bevorzugt umgibt die Detektorkammer den Detektor - bis auf die innere Fluidverbindung - gasdicht, also ohne optionale konstruktionsbedingte Öffnungen. Dank der Detektorkammer kann ein Gasgemisch ausschließlich oder im Wesentlichen nur durch die Modulatorkammer und die innere Fluidverbindung hindurch in die Detektorkammer fließen, aber nicht oder nur im geringen Maße direkt aus dem zu überwachenden Bereich in die Detektorkammer.

Möglich ist, dass die Modulatorkammer und die Detektorkammer in einem gemeinsamen Gehäuse angeordnet sind, welches zwei Öffnungen für die beiden Fluidverbindungen aufweist. Möglich ist, dass die Detektorkammer in einer Fluidverbindung mit einer weiteren Kammer der Gasdetektionsvorrichtung steht, wobei diese weitere Kammer aber ebenfalls kein oder nur wenig Gas aus der Umgebung in die Detektorkammer fließen lässt, weil die weitere Kammer gasdicht gegen den zu überwachenden Bereich abgedichtet ist.

Dann, wenn der Modulator eine hohe Temperatur aufweist, wird ein großer Teil des Zielgases in der Modulatorkammer oxidiert. Wie weiter oben bereits dargelegt, kann brennbares Zielgas im Wesentlichen nur durch die Modulatorkammer in die Detektorkammer gelangen. Daher befindet sich dann, wenn die Modulator-Temperatur einen hohen Temperatur-Wert aufweist, in der Detektorkammer nur wenig oder überhaupt kein brennbares Zielgas. Diese Wirkung tritt selbst dann ein, wenn der zu überwachende Bereich ein brennbares Zielgas enthält und dieses brennbare Zielgas durch die äußere Fluidverbindung in die Modulatorkammer gelangt. Die Detektorkammer verhindert, dass eine relevante Menge von brennbarem Zielgas den Modulator "umgeht" und aus dem Bereich direkt in die Detektorkammer fließt. Vielmehr kann ein Gasgemisch und damit ein brennbares Zielgas aus dem zu überwachenden Bereich im Wesentlichen nur durch die Modulatorkammer in die Detektorkammer gelangen. Wenn die Modulator-Temperatur einen hohen Temperatur-Wert aufweist, wird das Detektor-Bauteil daher im Wesentlichen nur von dem elektrischen Strom, der durch den Detektor fließt, erhitzt, egal ob sich in der Umgebung ein brennbares Zielgas befindet oder nicht. In der Detektorkammer ist dann kein oder nur wenig Zielgas vorhanden, welches der Detektor oxidieren könnte, egal ob in dem zu überwachenden Bereich ein solches brennbares Zielgas vorhanden ist oder nicht. Wenn der Modulator hingegen eine niedrige Temperatur aufweist, wird nur ein kleiner Teil des Zielgases oder sogar überhaupt kein Zielgas in der Modulatorkammer oxidiert. Bei einer niedrigen Modulator-Temperatur fließt daher eine große Menge von brennbarem Zielgas durch die innere Fluidverbindung in die Detektorkammer, vorausgesetzt der zu überwachende Bereich enthält ein brennbares Zielgas. Der erhitzte Detektor oxidiert wenigstens einen Teil dieses Zielgases in der Detektorkammer. Dadurch wird der Detektor weiter erhitzt, also zusätzlich zu der Erhitzung aufgrund der angelegten elektrischen Spannung. Die Temperatur des Detektors resultiert also bei einer geringen Modulator-Temperatur aus einer Überlagerung der Erhitzung, welche die angelegte Spannung bewirkt, und der Erhitzung, welche die Oxidation des Zielgases bewirkt.

Erfindungsgemäß oszilliert die Modulator-Temperatur zeitlich, und zwar in Folge der angelegten oszillierenden elektrischen Spannung. Die gerade beschriebene Wirkungskette hat zur Folge, dass die Konzentration eines brennbaren Zielgases in der Detektorkammer oszilliert und daher auch die Detektor-Temperatur und damit die Detektions-Größe oszilliert, wobei diese Detektions-Größe vom Oxidieren des Zielgases in der Detektorkammer beeinflusst wird.

Diese Oszillation der Detektor-Temperatur ist aufgrund der oben beschriebenen Wirkungskette synchron mit dem Oszillieren der Modulator-Temperatur und in der Regel auch synchron mit dem Oszillieren der am Modulator anliegenden elektrischen Spannung. Unter einem synchronen Oszillieren zweier Größen wird eine Oszillation verstanden, bei der die Frequenz der einen oszillierenden Größe um höchstens 50 %, bevorzugt höchstens um 20 %, von der Frequenz der anderen oszillierenden Größe abweicht. Die Amplituden der Oszillationen können sich stärker voneinander unterscheiden, und die beiden Oszillationen können zeitversetzt (phasenverschoben) auftreten.

Der oder mindestens ein Detektor-Sensor misst eine Detektions-Größe. Die Detektions-Größe ist die Detektor-Temperatur oder korreliert mit der Detektor-Temperatur und oszilliert daher ebenfalls. Diese Oszillation der Detektor-Temperatur und damit der Detektions-Größe tritt nur dann ein, wenn das Gasgemisch, welches durch die äußeren Fluidverbindung hindurch in die Modulatorkammer und von dort durch die innere Fluidverbindung hindurch in die Detektorkammer gelangt, eine ausreichend große Konzentration mindestens eines brennbaren Zielgases aufweist, und ansonsten nicht. Die Oszillation der Detektor-Temperatur und damit die Oszillation der Detektions-Größe werden durch die Oszillation der Modulator-Temperatur hervorgerufen und korrelieren mit der elektrischen Spannung, die am Modulator anliegt, und / oder mit der Stromstärke des durch den Modulator fließenden elektrischen Stroms. Eine Oszillation der Detektor-Temperatur und damit der Detektions-Größe, die von einer zeitlich veränderlichen Konzentration des brennbaren Zielgases in der Detektorkammer hervorgerufen wird, wird im Folgenden als "signifikante Oszillation" bezeichnet. Diese signifikante Oszillation bewirkt auch eine signifikante Oszillation der Detektions-Größe, welche der oder ein Detektor-Sensor misst. Falls eine signifikante Oszillation vorliegt, steht fest, dass ein brennbares Zielgas vorhanden ist. Falls keine signifikante Oszillation vorliegt, liegt auch kein brennbares Zielgas vor. Ein signifikantes Oszillieren lässt sich in aller Regel sicher von einem unvermeidlichen zeitlichen Rauschen der Detektions-Größe und von einer zeitlichen Veränderung der Konzentration des Zielgases in dem zu überwachenden Bereich unterscheiden.

Möglich ist, dass nur in einem Teil des Auswerte-Zeitraums ein synchrones Oszillieren stattfindet. Beispielsweise tritt ein brennbares Zielgas im Verlauf des Auswerte-Zeitraums in den zu überwachenden Bereich aus, es war zu Beginn des Auswerte-Zeitraums also noch nicht vorhanden. Außerdem verstreicht in manchen Fällen nach der Inbetriebnahme der erfindungsgemäßen Gasdetektionsvorrichtung eine Zeitspanne, bis ein Gasgemisch aus dem zu überwachenden Bereich in die Modulatorkammer und weiter in die Detektorkammer fließt, insbesondere wenn das Gasgemisch ausschließlich durch Diffundieren in die Detektorkammer gelangt, also nicht angesaugt wird. Daher beginnt ein synchrones Oszillieren in vielen Fällen erst eine gewisse Zeitspanne, nachdem die Gasdetektionsvorrichtung in Betrieb genommen wurde.

Der oder mindestens ein Detektor-Sensor misst die Detektions-Größe und daher direkt oder indirekt die Temperatur des Detektors, die vom Oxidieren des Zielgases erhöht wird und beim Fehlen von brennbarem Zielgas in der Detektorkammer wieder absinkt. In vielen Fällen vermag die Auswerteeinheit bereits aufgrund der Messwerte des oder eines Detektor-Sensors automatisch zu entscheiden, ob eine signifikante Oszillation vorliegt oder nicht. Der zeitliche Verlauf der Detektor-Temperatur und / oder der zeitliche Verlauf der am Detektor anliegenden elektrischen Spannung und / oder die Stromstärke am Detektor lassen sich messen und fungieren daher in einer Ausgestaltung als die Detektions-Größe.

Der Detektor-Sensor misst wiederholt die Detektions-Größe, also direkt die aktuelle Temperatur des Detektors oder einen sonstigen Detektor-Parameter, der mit der aktuellen Detektor-Temperatur korreliert, beispielsweise den elektrischen Widerstand. Die Auswerteeinheit empfängt Messwerte von diesem Detektor-Sensor, analysiert diese Messwerte und entscheidet aufgrund der Analyse automatisch, ob eine signifikante Oszillation der Detektor-Temperatur vorliegt oder nicht. Bei Vorliegen einer signifikanten Oszillation entscheidet die Auswerteeinheit automatisch, dass der zu überwachende Bereich mindestens ein brennbares Zielgas aufweist. Bevorzugt entscheidet die Auswerteeinheit beim Ausbleiben einer signifikanten Oszillation automatisch, dass der Bereich kein brennbares Zielgas aufweist.

Erfindungsgemäß oszilliert die Modulator-Temperatur, und zwar aufgrund der zeitlich veränderlichen Spannung, die am Modulator anliegt, und außerdem dadurch, dass Zielgas in der Modulatorkammer oxidiert wird. Die anliegende elektrische Spannung lässt sich messen und steuern oder regeln. Mindestens dann, wenn kein brennbares Zielgas vorhanden ist, oszilliert die Modulator-Temperatur daher in einer vorgegebenen oder messbaren oder regelbaren Weise. Eine Umgebungstemperatur, bei der die erfindungsgemäße Gasdetektionsvorrichtung eingesetzt wird, verändert sich in aller Regel wesentlich langsamer als die Modulator-Temperatur. Daher vermag die Auswerteeinheit in vielen Fällen sicher zu unterscheiden, ob die Detektor-Temperatur signifikant oszilliert oder eine auf andere Weise verursachte zeitliche Veränderung der Detektor-Temperatur vorliegt. Im ersten Fall ist ein brennbares Zielgas in dem zu überwachenden Bereich detektiert, im zweiten Fall weist der zu überwachende Bereich kein brennbares Zielgas oberhalb einer Nachweisgrenze auf.

Die erfindungsgemäße Gasdetektionsvorrichtung detektiert ein brennbares Zielgas mit großer Zuverlässigkeit. Ob ein Zielgas detektiert wird oder nicht, hängt vom signifikanten Oszillieren ab und nicht von der Lage eines Nullpunkts oder Referenzpunkts der Detektions-Größe, der für einen Zustand frei von Zielgas steht.

Insbesondere deshalb, weil sich ein signifikantes Oszillieren meistens sicher detektieren oder sicher ausschließen lässt, ist die erfindungsgemäße Gasdetektionsvorrichtung relativ unempfindlich gegenüber Schwankungen von Umgebungsbedingungen, bei denen die erfindungsgemäße Gasdetektionsvorrichtung eingesetzt wird. Mit anderen Worten: Das signifikante Oszillieren tritt auch bei sich verändernden Umgebungsbedingungen auf, insbesondere bei sich verändernden Umgebungstemperaturen, und zwar in der Regel dann und nur dann, wenn ein brennbares Zielgas vorhanden ist. Dieser Vorteil ist insbesondere dann von Bedeutung, wenn die Gasdetektionsvorrichtung außerhalb eines Gebäudes eingesetzt wird und daher der zu überwachende Bereich daher dem Wetter ausgesetzt ist. In vielen Fällen erspart die Erfindung die Notwendigkeit, einen Sensor für eine Umgebungstemperatur oder eine sonstige Umgebungsbedingung zu verwenden oder die Umgebungsbedingung der Gasdetektionsvorrichtung vorzugeben. Vielmehr liefert die erfindungsgemäße Gasdetektionsvorrichtung auch bei unbekannten Umgebungsbedingungen häufig zuverlässig Ergebnisse.

Die Gasdetektionsvorrichtung vermag nach Ablauf eines Zeitintervalls sicher zu entscheiden, ob ein brennbares Zielgas in dem Bereich vorhanden ist oder nicht. In der Regel umfasst dieses Zeitintervall nur wenige Schwankungen der Modulator-Temperatur, manchmal nur eine einzige Schwankung von der minimalen zur maximalen und zurück zur minimalen Modulator-Temperatur. Insbesondere dann, wenn das Gasgemisch ausreichend rasch in das Innere der Gasdetektionsvorrichtung und damit in die Modulatorkammer diffundiert oder eingesaugt wird, vermag die erfindungsgemäße Gasdetektionsvorrichtung daher in vielen Fällen ausreichend schnell genug und zuverlässig genug einen Alarm auszulösen, wenn ein brennbares Zielgas in dem zu überwachenden Bereich aufgetreten ist, und dies, obwohl der Durchfluss und damit die Durchflussrate in die Detektorkammer erfindungsgemäß limitiert ist.

Wie bereits beschrieben, ist ein Zielgas dann detektiert, wenn die Auswerteeinheit ein signifikantes Oszillieren festgestellt hat. In vielen Fällen führt auch eine relativ geringe Konzentration eines brennbaren Zielgases in der Umgebung der Gasdetektionsvorrichtung zu diesem signifikanten Oszillieren. Daher vermag die erfindungsgemäße Gasdetektionsvorrichtung in vielen Fällen auch eine relativ geringe Konzentration eines brennbaren Zielgases sicher zu detektieren, wenn ein signifikantes Oszillieren detektiert ist. Eine herkömmliche Gasdetektionsvorrichtung vermag eine so geringe Konzentration häufig gar nicht zu detektieren. Die erfindungsgemäße Gasdetektionsvorrichtung weist daher eine niedrigere Nachweisgrenze als manche herkömmliche Gasdetektionsvorrichtungen auf. Andererseits ist das Ausbleiben eines signifikanten Oszillierens ein relativ sicherer Nachweis, dass kein brennbares Zielgas in der Umgebung vorhanden ist. Die erfindungsgemäße Gasdetektionsvorrichtung führt daher relativ selten zu Fehlalarmen, d.h. sie detektiert in relativ wenigen Fällen fälschlicherweise das Vorhandensein eines brennbaren Zielgases. Die erfindungsgemäße Gasdetektionsvorrichtung vermag in ausreichend kurzer Zeit und mit relativ hoher Zuverlässigkeit eine Meldung, dass der Bereich frei von Zielgas ist, zu generieren.

In vielen Fällen verändert der Detektor sich im Verlaufe des Einsatzes, insbesondere aus den folgenden Gründen: Erfindungsgemäß fließt Strom durch den Detektor, wodurch das Detektor-Bauteil erhitzt wird. Außerdem wird in der Umgebung des Detektors ein Zielgas oxidiert, und die freiwerdende Wärmeenergie erhitzt von außen den Detektor. Weil Wärmeenergie sowohl im Detektor erzeugt wird, nämlich weil eine elektrische Spannung anliegt und Strom fließt, als auch von außen auf den Detektor einwirkt, nämlich durch die Oxidation, kann der Detektor physikalisch verändert werden. Diese physikalische Veränderung kann Einfluss auf die Detektions-Größe und damit auf ein Signal nehmen, welches zum Detektieren des Zielgases ausgewertet wird. Darüber hinaus kann das Gasgemisch, welches durch die Modulatorkammer in die Detektorkammer gelangt, mindestens eine Substanz umfassen, welche den erhitzten Detektor chemisch verändert, beispielsweise eine chemisch aggressive oder oxidierende oder korrodierende Substanz.

Aus den gerade genannten Gründen verändert sich der Detektor in der Regel im Laufe der Zeit in der Regel zwangsläufig, und zwar vor allem chemisch und oft auch physikalisch. Diese insbesondere chemische Veränderung kann sich auf die Detektions-Größe auswirken, welche der Detektor-Sensor zu messen vermag. Insbesondere kann der kausale Zusammenhang zwischen dem Oxidieren des Zielgases und dem Erhitzen des Detektors sich im Laufe der Zeit verändern, weil der Detektor sich verändert ("altert"). Außerdem kann die maximale elektrische Spannung, die am Detektor und am Modulator anliegt, im Laufe der Zeit abnehmen, insbesondere wenn die Gasdetektionsvorrichtung eine eigene Spannungsversorgungseinheit aufweist und damit unabhängig von einem stationären Stromversorgungsnetz eingesetzt werden kann.

Die erfindungsgemäße Gasdetektionsvorrichtung ist relativ unempfindlich gegenüber einer solchen Alterung oder sonstigen zeitlichen Veränderung des Detektors, vor allem weil sich ein signifikantes Oszillieren unabhängig von einer Veränderung des Detektors in vielen Fällen automatisch sicher erkennen oder sicher ausschließen lässt. In vielen Fällen ist es daher nicht erforderlich, den Detektor häufig zu justieren, auch wenn er sich im Laufe der Zeit verändert. Dieser Vorteil ist insbesondere dann wichtig, wenn ein großer Bereich mittels einer Vielzahl von Gasdetektionsvorrichtungen auf mindestens ein brennbares Zielgas überwacht werden soll. Die Erfindung verringert den Aufwand, der zur Wartung dieser Gasdetektionsvorrichtungen erforderlich ist.

Ein Problem vieler bekannter Gasdetektionsvorrichtungen ist, dass die Gasdetektionsvorrichtung und insbesondere der Detektor im Laufe der Zeit "vergiftet" wird, insbesondere weil das Erhitzen des Detektors und die beim Oxidieren freiwerdende Wärmeenergie die Detektor-Temperatur und damit die Detektions-Größe im Laufe der Zeit verändern und / oder weil bestimmte Zielgases mit dem Detektor chemisch reagieren. Auch der Detektor und der Modulator der erfindungsgemäßen Gasdetektionsvorrichtung können im Laufe der Zeit zunehmend "vergiftet" werden.

Die erfindungsgemäße Gasdetektionsvorrichtung weist trotzdem in vielen Fällen eine höhere Resistenz gegenüber Gasen auf, welche den Detektor und / oder den Modulator vergiften können. Diese gewünschte Wirkung tritt insbesondere aus folgendem Grund ein: Der Modulator wird erfindungsgemäß dergestalt mit elektrischer Spannung versorgt, dass die Modulator-Temperatur oszilliert, insbesondere wird der Modulator gepulst betrieben. Wenn das Modulator-Bauteil auf eine hohe Temperatur erhitzt ist, oxidiert der Modulator in der Modulatorkammer Zielgas, und weniger brennbares Zielgas fließt in die Detektorkammer, sodass der Detektor nicht der Wärmeenergie ausgesetzt ist, welche ansonsten durch die Oxidation freigesetzt werden würde. Nur dann, wenn das Modulator-Bauteil auf eine geringe Temperatur erhitzt wird oder der Modulator sogar ausgeschaltet ist, kann ein brennbares Zielgas durch die Modulatorkammer hindurch zum Detektor gelangen und vom Detektor oxidiert werden. In manchen Fällen reduziert der eingeschaltete und erhitzte Modulator auch den Fluss eines schädlichen und nicht oxidierten Gases in die Detektorkammer. Ein möglicherweise schädliches Gas wird also auf zwei Bestandteile der Gasdetektionsvorrichtung verteilt, nämlich auf den Detektor und den Modulator. Der Detektor ist schädlichen Gasen weniger ausgesetzt. Bei einer herkömmlichen Gasdetektionsvorrichtung ist der Detektor hingegen dauerhaft dem Zielgas ausgesetzt und oxidiert dauerhaft das Zielgas.

Zwar ist der erfindungsgemäße Modulator dauerhaft einem schädlichen Gas ausgesetzt. Jedoch wird ein Zielgas anhand der signifikanten Oszillation erkannt, also nicht oder nicht nur aufgrund eines einzelnen Werts eine Detektions-Größe. Dank der Erfindung wird nicht notwendigerweise eine Detektions-Größe des Modulators gemessen und für die Detektion verwendet. Die erfindungsgemäße Gasdetektionsvorrichtung ist daher weniger empfindlich gegenüber einer Vergiftung des Modulators.

Eine mögliche Abhilfe, um zu verhindern, dass der Detektor durch ein schädliches Gas vergiftet wird, ist die folgende: Die äußere Fluidverbindung wird mit einem Filter versehen, wobei dieser Filter das Eindringen von Gasen, die für den Detektor schädlich sind, in die Detektorkammer verhindert. Die Erfindung ermöglicht es, vermeidet aber die Notwendigkeit, die äußere und / oder die innere Fluidverbindung mit einem Filter zu versehen, welcher das Eindringen von bestimmten Zielgasen in die Modulatorkammer und weiter in die Detektorkammer verhindert. Ein Nachteil eines solchen Filters ist der folgende: Ein solcher Filter könnte dazu führen, dass der Detektor ein brennbares Zielgas nicht detektiert, obwohl dieses Zielgas in der Umgebung der Gasdetektionsvorrichtung vorhanden ist und detektiert werden sollte. Dieses unerwünschte Ergebnis kann auftreten, weil das Zielgas aufgrund des Filters überhaupt nicht oder nur sehr langsam in die Detektorkammer gelangt.

Die erfindungsgemäße Gasdetektionsvorrichtung erfordert keine beweglichen mechanischen Bauteile, insbesondere keine oszillierenden mechanischen Bauteile. Ein solches bewegliches mechanisches Bauteil verschleißt zwangsläufig während des Betriebs. Insbesondere kann eine Lagerung verschleißen oder blockieren, wobei ein mechanisches Bauteil in dieser Lagerung beweglich gelagert ist. Ein mechanisches Bauteil kann beschädigt werden, wenn die Gasdetektionsvorrichtung einer Erschütterung oder einem sonstigen mechanischen Impuls ausgesetzt ist. Außerdem ist es erforderlich, ein bewegliches mechanisches Bauteil zu überwachen, um den Verschleiß und den Ausfall zu detektieren. Die erfindungsgemäße Gasdetektionsvorrichtung benötigt weder ein bewegliches mechanisches Bauteil noch eine Lagerung noch einen Sensor zur Überwachung eines verschleißenden mechanischen Bauteils und auch kein Stellglied zum aktiven Bewegen eines beweglichen Bauteils. Daher hat die erfindungsgemäße Gasdetektionsvorrichtung in vielen Fällen eine längere Lebensdauer und / oder ein geringeres Gewicht und / oder einen geringeren Energieverbrauch als andere Gasdetektionsvorrichtungen.

Die erfindungsgemäße Gasdetektionsvorrichtung benötigt in vielen Fällen keine Pumpe oder sonstige Fördereinrichtung, welche ein zu untersuchendes Gasgemisch zu der Detektorkammer fördert. Sowohl die äußere als auch die innere Fluidverbindung können einen ausreichend großen Durchmesser aufweisen, sodass ein zu untersuchendes Gasgemisch allein durch Diffusion rasch und zuverlässig in die Detektorkammer gelangt. Auch dieser Vorteil wird vor allem deshalb erzielt, weil die Auswerteeinheit ein brennbares Zielgas aufgrund eines signifikanten Oszillierens entdeckt und nicht durch einen Vergleich mit einem Referenzwert, der für ein Gasgemisch gewonnen wird, das frei von einem brennbaren Zielgas ist.

Einige aus dem Stand der Technik bekannte Gasdetektionsvorrichtungen umfassen zusätzlich zu dem Detektor eine Referenzeinheit, beispielsweise einen Kompensator. Ein Signal dieser Referenzeinheit ermöglicht es, bis zu einem gewissen Grade den Einfluss von Umgebungsbedingungen auf die Detektions-Größe rechnerisch zu kompensieren. Beim Einsatz einer solchen aus dem Stand der Technik bekannten Gasdetektionsvorrichtung ist der Detektor von einem Gasgemisch umgeben, welches ein brennbares Zielgas enthalten kann, während die Referenzeinheit in einer Ausgestaltung von einem Gasgemisch umgeben ist, welches kein brennbares Zielgas enthält. In einer anderen aus dem Stand der Technik bekannten Ausführungsform wird die Referenzeinheit dem gleichen Gasgemisch wie der Detektor ausgesetzt, vermag aber nicht, ein brennbares Zielgas zu oxidieren. Sowohl die Detektoreinheit als auch die Referenzeinheit werden erhitzt, beispielsweise indem eine elektrische Spannung angelegt wird, und eine Detektions-Größe des Detektors wird mit der entsprechenden Detektions-Größe der Referenzeinheit verglichen. Ein signifikanter Unterschied zeigt an, dass ein Zielgas vorhanden ist.

Eine derartige Gasdetektionsvorrichtung mit Detektor und Referenzeinheit setzt voraus, dass der Detektor und die Referenzeinheit ausreichend ähnlich aufgebaut sind und sich die dennoch vorhandenen Unterschiede ausreichend gut rechnerisch kompensieren lassen. Nur dann lässt sich durch Vergleich der Werte, welche die Detektions-Größe für den Detektor bzw. die Referenzeinheit annimmt, sicher unterscheiden, ob das Gasgemisch ein brennbares Zielgas enthält oder nicht. Die erfindungsgemäße Gasdetektionsvorrichtung kann eine Referenzeinheit umfassen. Die Erfindung erspart aber die Notwendigkeit, eine Referenzeinheit vorzusehen, welche dem Detektor ausreichend ähnlich ist. Die Aufgabe des erfindungsgemäßen Modulators ist, Zielgas in der Modulatorkammer abwechselnd zu oxidieren und nicht zu oxidieren, sodass der Detektor dieses Zielgas abwechselnd nicht oxidiert und oxidiert. Der Modulator braucht nicht notwendigerweise auf die gleiche Weise oder im gleichen Umfang wie der Detektor Zielgas zu oxidieren oder die gleichen thermischen Eigenschaften aufzuweisen. Die Erfindung ermöglicht es, erfordert aber nicht, dass auch für den Modulator die Detektions-Größe gemessen wird.

Möglich ist, dass ein Modulator-Sensor eine Detektions-Größe des Modulators misst, die mit der Temperatur des Modulators korreliert, beispielsweise die Temperatur selbst oder die elektrische Spannung, die am Modulator anliegt, und / oder die Stromstärke des durch den Modulator fließenden Stroms. Bekanntlich korreliert der elektrische Widerstand mit der Temperatur. Indem Messwerte des Detektor-Sensors und optional eines weiteren Sensors, der eine Detektions-Größe des Modulators misst, ausgewertet werden, lässt sich in vielen Fällen sicher entscheiden, ob eine Oszillation der Detektor-Temperatur durch ein brennbares Zielgas in der Detektorkammer hervorgerufen wird, also eine signifikante Oszillation ist, oder durch sich verändernde Umgebungsbedingungen, insbesondere durch eine Veränderung der Umgebungstemperatur, und / oder durch ein unvermeidliches Messrauschen oder Prozessrauschen. Die Modulator-Temperatur hängt idealerweise nur von der am Modulator anliegenden elektrischen Spannung und der Konzentration eines brennbaren Zielgases ab, in der Praxis außerdem von Umgebungsbedingungen und einem unvermeidlichen Altern des Modulators. Dies gilt entsprechend für die Detektor-Temperatur. In vielen Fällen lässt sich die gemessene Modulator-Temperatur dafür verwenden, um den Einfluss von Umgebungsbedingungen und Altern auf die Detektor-Temperatur und damit auf die Detektions-Größe rechnerisch zu kompensieren. Außerdem ist es in manchen Fällen möglich, beim Ausfall des Detektors mithilfe der gemessenen Modulator-Temperatur ein brennbares Zielgas zu detektieren. In diesem Fall arbeitet der Modulator bevorzugt wie ein Detektor einer herkömmlichen Gasdetektionsvorrichtung.

Erfindungsgemäß oszilliert die elektrische Spannung, die an den Modulator angelegt wird. In vielen Fällen nimmt der Modulator dank der Oszillationen weniger elektrische Energie auf, als wenn der Modulator dauerhaft auf eine Temperatur erhitzt würde, die ausreichend hoch zum Oxidieren ist. Verglichen mit herkömmlichen Gasdetektionsvorrichtungen spart die erfindungsgemäße Gasdetektionsvorrichtung somit in vielen Fällen elektrische Energie ein.

Falls der zu überwachende Bereich ein brennbares Zielgas aufweist, oszilliert die durch das Oxidieren erhöhte Temperatur des Detektors und damit die Detektions-Größe auf eine signifikante Weise. In manchen Fällen unterscheidet sich ein Parameter dieser Oszillation von Zielgas zu Zielgas. Beispielsweise hängt der zeitliche Verlauf der Detektor-Temperatur und damit der zeitliche Verlauf der Detektions-Größe in einem Zeitintervall, in dem der Modulator eine niedrige Temperatur aufweist und daher nur wenig Zielgas zu oxidieren vermag, oder der Verlauf im Auswerte-Zeitraum in charakteristischer Weise von der Art und / oder der Konzentration des brennbaren Zielgases ab. Daher vermag die erfindungsgemäße Gasdetektionsvorrichtung in vielen Fällen nicht nur automatisch zu entscheiden, ob ein brennbares Zielgas in dem Bereich vorhanden ist oder nicht, sondern zusätzlich, von welcher Art dieses brennbare Zielgas ist und / oder in welcher Konzentration es vorliegt. In manchen Fällen lässt sich daher dieselbe erfindungsgemäße Gasdetektionsvorrichtung zum Detektieren von unterschiedlichen vorgegebenen Zielgasen verwenden.

Erfindungsgemäß ist ein Auswerte-Zeitraum vorgegeben. Mindestens in diesem Auswerte-Zeitraum oszilliert die Temperatur des Detektors, die durch das Anlegen der Spannung verursacht wird, überhaupt nicht oder mit einer geringeren Amplitude als die Temperatur des Modulators. Die Auswerteeinheit verwendet Messwerte, welche der Detektor-Sensor im Auswerte-Zeitraum erzeugt hat, um über das Vorhandensein oder Nichtvorhandensein des Zielgases zu entscheiden. Dieser Auswerte-Zeitraum kann gleich dem gesamten Einsatz-Zeitraum sein, in welchem die Gasdetektionsvorrichtung verwendet wird, oder ein Zeitraum, der kürzer als der Einsatz-Zeitraum ist. Der Auswerte-Zeitraum kann eine Abfolge von Auswerte-Intervallen umfassen, wobei zwischen zwei aufeinanderfolgenden Auswerte-Intervallen eine Lücke und somit ein zeitlicher Abstand auftritt. Die Ausgestaltung mit mehreren voneinander beabstandeten Auswerte-Intervallen spart in vielen Fällen elektrische Energie ein. Der Abstand zwischen zwei Auswerte Intervallen lässt sich abhängig von einer gewünschten Reaktionszeit der Gasdetektionsvorrichtung auf ein brennbares Zielgas festlegen.

In einer bevorzugten Ausgestaltung wird der Detektor mit einer gepulsten elektrischen Spannung versorgt, besonders bevorzugt auch der Modulator. Verglichen mit einer Ausgestaltung, bei welcher dauerhaft eine elektrische Spannung an dem Detektor anliegt, spart diese Ausgestaltung elektrische Energie ein. Die Frequenz und die Zeitdauer der elektrischen Pulse lässt sich dergestalt vorgeben, dass einerseits ein brennbares Zielgas rasch genug detektiert wird, was eine ausreichend große Pulsfrequenz erfordert, und andererseits der Verbrauch an elektrischer Energie relativ niedrig gehalten wird. Die Anforderung, Energie einzusparen, ist insbesondere dann wichtig, wenn die Gasdetektionsvorrichtung eine eigene Spannungsversorgungseinheit umfasst und überhaupt nicht oder nicht dauerhaft mit einem stationären Spannungsversorgungsnetz verbunden ist. Bevorzugt weisen die elektrischen Pulse der am Detektor anliegenden elektrischen Spannung eine geringere Amplitude und bevorzugt auch eine geringere Frequenz auf als die Pulse der am Modulator anliegenden elektrischen Spannung.

Falls die Spannung gepulst am Detektor anliegt, so wird bevorzugt als Auswerte-Zeitraum eine Abfolge von Zeitintervallen gewählt, in denen die anliegende elektrische Spannung oberhalb einer absoluten oder relativen Schranke liegt. Bevorzugt berücksichtigt die Auswerteeinheit nur dann einen Messwert des Detektor-Sensors, wenn dieser Messwert in einer Zeitspanne mit einer Spannung oberhalb der Schranke erzeugt worden ist.

Erfindungsgemäß umgibt die Modulatorkammer den Modulator und die Detektorkammer den Detektor. Die beiden Kammern reduzieren das Risiko, dass der erhitzte Modulator oder der erhitzte Detektor ein brennbares Zielgas außerhalb der Gasdetektionsvorrichtung entflammt oder gar zur Explosion bringt, was in der Regel unerwünscht und häufig sogar gefährlich ist. Umgekehrt schützen die Kammern den Modulator und den Detektor vor unerwünschten mechanischen und chemischen Umgebungseinflüssen. Bevorzugt ist die äußere Fluidverbindung mit einer Flammensperre oder einem sonstigen Flammschutz versehen, sodass das Risiko verringert wird, dass Flammen aus der Modulatorkammer nach außen schlagen.

Erfindungsgemäß umgibt die Modulatorkammer den Modulator und weist die äußere Fluidverbindung auf. In einer Ausgestaltung trennt die Modulatorkammer den Modulator fluiddicht von der Umgebung, mit Ausnahme der äußeren Fluidverbindung. Die Detektorkammer umgibt den Detektor fluiddicht, mit Ausnahme der inneren Fluidverbindung. Diese Ausgestaltung reduziert weiter das Risiko für das unerwünschte Ereignis, dass eine große Menge eines Gasgemischs mit einem brennbaren Zielgas den Modulator umgeht und in die Detektorkammer gelangt, ohne dass der erhitzte Modulator das Zielgas oxidieren kann. Falls Zielgas den Modulator umgeht, könnte die Gasdetektionsvorrichtung falsche Detektions-Ergebnisse erzielen.

Erfindungsgemäß wird an den Modulator eine elektrische Spannung dergestalt angelegt, dass die Temperatur des Modulators oszilliert. In einer Ausgestaltung wechselt die Modulator-Temperatur zwischen einem niedrigeren Temperatur-Wert und einem höheren Temperatur-Wert, vorzugsweise in Form eines zeitlichen Rechteck-Verlaufs. Der niedrige Temperatur-Wert wird beispielsweise durch einen spannungslosen Zustand erzielt.

In einer bevorzugten Ausgestaltung wird ein gewünschter oder geforderter zeitlicher Verlauf der Modulator-Temperatur vorgegeben, beispielsweise ein Rechteck-Verlauf. Ein Modulator-Temperatur-Sensor misst wiederholt ein Maß für die tatsächliche aktuelle Modulator-Temperatur. Beispielsweise misst der Modulator-Temperatur-Sensor direkt die Modulator-Temperatur oder auch den elektrischen Widerstand des Modulators. Ein signalverarbeitendes Steuergerät regelt automatisch die Modulator-Temperatur. Bei dieser Regelung ist der vorgegebene gewünschte zeitliche Verlauf des Maßes für die Modulator-Temperatur die Führungsgröße, und der gemessene tatsächliche zeitliche Verlauf des Maßes für die Modulator-Temperatur ist die Regelgröße. Als die oder eine Stellgröße der Regelung fungiert bevorzugt die elektrische Spannung, die am Modulator anliegt und die veränderlich ist. Das Steuergerät verändert diese Spannung. Als eine Stellgröße kann auch die elektrische Stromstärke oder eine sonstige veränderbare und steuerbare elektrische Größe fungieren. Die Konzentration eines brennbaren Zielgases sowie die Umgebungsbedingungen sind mögliche Störgrößen für diese Regelung.

Diese Ausgestaltung, dass die Modulator-Temperatur geregelt wird, führt dazu, dass das thermische Verhalten und der thermische Haushalt des Modulators im Wesentlichen von dem vorgegebenen zeitlichen Verlauf der gewünschten oder geforderten Modulator-Temperatur abhängt und nur in relativ geringem Maße von der Konzentration von brennbarem Zielgas in dem zu überwachenden Bereich oder in der Modulatorkammer. Diese Ausgestaltung erleichtert es, die Gasdetektionsvorrichtung auszulegen und ihre Lebensdauer vorherzusagen.

In der gerade beschriebenen Ausgestaltung wird die Temperatur des Modulators als eine regelbare Größe verwendet. Anstelle der Temperatur lässt sich auch eine andere regelbare Größe verwenden, die mit der Temperatur korreliert und die von einem Modulator-Regelgrößen-Sensor gemessen wird, beispielsweise
- der elektrische Widerstand des Modulators, der bekanntlich von der Temperatur abhängt,
- die Stärke des elektrischen Stroms, welche durch den Modulator fließt,
- die elektrische Spannung, welche am Modulator anliegt, oder
- die vom Modulator aufgenommene elektrische Leistung.

Auch bei diesen Ausgestaltungen wird bevorzugt die elektrische Spannung als die Stellgröße verwendet, d. h. die am Modulator anliegende elektrische Spannung wird mit dem Regelungsziel verändert, dass die regelbare Größe des Modulators einem gewünschten zeitlichen Verlauf folgt. Möglich ist auch, die Stärke des durch den Modulator fließenden Stroms als die Stellgröße zu verwenden.

Erfindungsgemäß wird an den Detektor eine elektrische Spannung angelegt. Das Anlegen dieser Spannung bewirkt, dass elektrischer Strom durch den Detektor fließt und das Detektor-Bauteil sich erwärmt. Die Temperatur, die durch die anliegende Spannung verursacht wird, oszilliert erfindungsgemäß mit geringerer Amplitude als die Modulator-Temperatur, optional überhaupt nicht. Die Detektor-Temperatur wird einerseits von dem elektrischen Strom, der durch den Detektor fließt, und andererseits durch das Oxidieren von brennbarem Zielgas in der Detektorkammer beeinflusst.

In einer bevorzugten Ausgestaltung wird ein gewünschter oder geforderter zeitlicher Verlauf der Stromstärke des durch den Detektor fließenden Stroms vorgegeben. Ein Stromstärken-Sensor misst wiederholt die tatsächliche Stromstärke. Ein Steuergerät regelt die Stromstärke des durch den Detektor fließenden Stroms. Bei dieser Regelung ist der vorgegebene gewünschte zeitliche Verlauf der Stromstärke die Führungsgröße, und der gemessene zeitliche Verlauf der tatsächlichen Stromstärke ist die Regelgröße. Als die oder eine Stellgröße fungiert bevorzugt die elektrische Spannung, die am Detektor anliegt und die zeitlich veränderlich ist. Das Steuergerät verändert diese anliegende elektrische Spannung. Wiederum sind die Konzentration eines brennbaren Zielgases und Umgebungsbedingungen mögliche Störgrößen.

Diese Ausgestaltung erhöht die Zuverlässigkeit, mit der das Vorhandensein eines brennbaren Zielgases sicher von dessen Nicht-Vorhandensein unterschieden wird. Falls in dem zu überwachenden Bereich kein brennbares Zielgas vorhanden ist und daher auch kein brennbares Zielgas in die Detektorkammer gelangt, hängt der zeitliche Verlauf der tatsächlichen Detektor-Temperatur im Wesentlichen von dem zeitlichen Verlauf der Stromstärke des durch den Detektor fließenden Stroms und der anliegenden Spannung ab. Dieser zeitliche Stromstärken-Verlauf und dieser zeitliche Spannungs-Verlauf sind durch die Regelung ausreichend genau bekannt. Durch die Konstruktion des Detektors, insbesondere durch dessen elektrischen Widerstand, ist in vielen Fällen auch ausreichend genau bekannt, welchen zeitlichen Verlauf die Detektor-Temperatur hat, falls kein brennbares Zielgas in der Detektorkammer vorhanden ist. Ein Zielgas ist vorhanden, wenn folgendes Ereignis detektiert wird: Die Detektor-Temperatur und damit die Detektions-Größe oszillieren, und dieses Oszillieren unterscheidet sich signifikant von dem zeitlichen Verlauf der Auswirkung, die der durch den Detektor fließende elektrische Strom auf die Detektor-Temperatur hat.

Wie gerade beschrieben wird in einer Ausgestaltung ein gewünschter oder geforderter zeitlicher Verlauf der Stromstärke vorgegeben. Eine Realisierungsform dieser Ausgestaltung ist die Vorgabe, dass diese Stromstärke zeitlich konstant bleiben soll. Die Regelung wird also mit dem Ziel durchgeführt, dass die Stromstärke des durch den Detektor fließenden Stroms zeitlich konstant bleiben soll. Das Oxidieren von brennbarem Zielgas in der Detektorkammer verändert in vielen Fällen den elektrischen Widerstand des Detektors. Bekanntlich steigt der elektrische Widerstand von vielen Metallen, z.B. Platin, mit der Temperatur. Dieser Effekt wird dadurch kompensiert, dass eine Regelung der Stromstärke durchgeführt und bei Bedarf die anliegende elektrische Spannung verändert wird. Falls die Detektor-Temperatur und damit die Detektions-Größe trotz der Regelung oszilliert, so wurde mit relativ hoher Sicherheit mindestens ein brennbares Zielgas in der Detektorkammer und damit in dem zu überwachenden Bereich detektiert.

In der gerade beschriebenen Ausgestaltung fungiert die Stromstärke als die regelbare Größe des Detektors. Möglich ist auch, eine andere Größe des Detektors zu regeln, die mit der Temperatur korreliert und die von der Detektions-Größe verschieden ist, beispielsweise eine der folgenden Größen:
- die am Detektor anliegende elektrische Spannung,
- der elektrische Widerstand des Detektors,
- die vom Detektor aufgenommene elektrische Leistung,
- direkt die Temperatur des Detektors.

Ein Detektor-Regelgrößen-Sensor misst diese Größe, die mit der Temperatur korreliert und die geregelt wird. Auch bei diesen Ausgestaltungen wird bevorzugt die elektrische Spannung als die Stellgröße verwendet, d. h. die am Detektor anliegende elektrische Spannung wird mit dem Regelungsziel verändert, dass die regelbare Größe des Detektors einem gewünschten zeitlichen Verlauf folgt. Dies gilt natürlich nur dann, wenn die anliegende elektrische Spannung nicht die Detektions-Größe ist. Möglich ist auch, die Stärke des durch den Detektor fließenden Stroms als die Stellgröße zu verwenden.

In vielen Fällen wird die Kenntnis eines sogenannten Nullpunkts des Detektors gewünscht. Dieser Nullpunkt ist der aktuelle Wert der Detektions-Größe in einer Situation, in der kein Zielgas in der Detektorkammer oxidiert wird. Weil der Detektor sich im Laufe der Zeit durch Alterung ändert und auch die Umgebungsbedingungen veränderlich sein können, driftet der Detektor-Nullpunkt in der Regel im Laufe der Zeit, bleibt also nicht konstant. Eine Ausgestaltung der Erfindung ermöglicht es, den Nullpunkt automatisch zu bestimmen, und zwar bevorzugt erneut bei jedem Messzyklus. Dadurch wird der Nullpunkt automatisch aktualisiert, auch bei einer allmählichen Veränderung des Detektors.

Gemäß dieser Ausgestaltung wird an den Modulator eine elektrische Spannung dergestalt angelegt, dass die tatsächliche Modulator-Temperatur zwischen einem minimalen Temperatur-Wert und einem maximalen Temperatur-Wert oszilliert, beispielsweise mit einem rechteckigen oder dreieckigen oder sinusförmigen Verlauf. Bevorzugt wird die gerade beschriebene Regelung der Modulator-Temperatur oder einer sonstigen regelbaren Modulator-Größe durchgeführt. Bevorzugt werden der maximale Temperatur-Wert und das Zeitintervall, in dem der Modulator eine Temperatur oberhalb einer vorgegebenen Temperatur-Schranke aufweist, so vorgegeben, dass der Modulator dann, wenn er den maximalen Temperatur-Wert aufweist, mit großer Sicherheit praktisch das gesamte brennbares Zielgas in der Modulatorkammer oxidiert. In diesem Zeitintervall gelangt praktisch kein brennbares Zielgas in die Detektorkammer. In der Detektorkammer kann daher nicht durch eine Oxidation eines brennbaren Zielgases Wärmeenergie freigesetzt werden, und zwar auch dann nicht, wenn der zu überwachende Bereich ein brennbares Zielgas enthält. In diesem Zeitintervall, in dem die Detektorkammer frei von einem brennbaren Zielgas ist, misst der oder ein Detektor-Sensor mindestens einmal die Detektions-Größe. Der in diesem Zeitintervall gemessene Wert der Detektions-Größe wird als der gesuchte Nullpunkt verwendet.

Diese Ausgestaltung, den Nullpunkt festzulegen, erspart die Notwendigkeit, in einem Bereich außerhalb der Gasdetektionsvorrichtung einen Zustand frei von einem Zielgas herzustellen und dann zu messen. Der Nullpunkt lässt sich vielmehr unabhängig von der Konzentration eines brennbaren Zielgases in dem zu überwachenden Bereich festlegen, weil der Modulator bewirkt, dass in der Detektorkammer kein brennbares Zielgas vorhanden ist.

In einer bevorzugten Ausgestaltung umfassen sowohl der Detektor als auch der Modulator jeweils
- einen Draht aus einem elektrisch leitenden Material, insbesondere aus Platin oder aus einer Legierung, die Platin enthält, wobei dieser Draht als das erhitzte Bauteil fungiert,
- eine Isolierung, welche den Draht elektrisch isoliert, aber nicht thermisch, insbesondere eine Isolierung in Form einer keramischen Ummantelung, und
- ein katalytisches Material in oder außen auf der elektrischen Isolierung, insbesondere eine Beschichtung der äußeren Oberfläche der Isolierung aus einem katalytischen Material und / oder ein katalytisches Material, welches in die Isolierung eingebettet ist.

Der elektrisch leitende Draht umfasst ein heizendes Segment, beispielsweise eine Spule aus aufgewickeltem Draht. Dieses heizende Segment erhitzt sich, wenn eine elektrische Spannung an den Draht angelegt wird und daher Strom durch den Draht fließt. Die elektrische Isolierung des Drahts ist bevorzugt aus einem keramischen Material hergestellt und hat beispielsweise die Form einer Kugel oder eines Ellipsoids. Dies kann für den Draht des Detektors und / oder für den Draht des Modulators gelten.

Besonders bevorzugt sind sowohl der Detektor als auch der Modulator jeweils als ein Pellistor ausgestaltet.

In vielen Fällen ist es dank des katalytischen Materials möglich, den Detektor und den Modulator mit einer niedrigeren Temperatur zu betreiben als bei einer denkbaren Ausgestaltung ohne katalytisches Material. Trotz der niedrigeren Temperatur wird dank des katalytischen Materials ein brennbares Zielgas oxidiert. Dank der niedrigeren Temperatur wird das Zielgas in vielen Fällen oxidiert, ohne zu verbrennen oder gar zu explodieren, und elektrische Energie wird eingespart. Bei einer sehr hohen Konzentration von brennbarem Zielgas kann aber ein Zielgas mit offener Flamme verbrennen oder gar explodieren, obwohl der Detektor und / oder der Modulator keramisches Material aufweisen.

Die erfindungsgemäße Gasdetektionsvorrichtung kann als ein stationäres oder auch als ein tragbares Gerät ausgestaltet sein. In einer Ausgestaltung besitzt die Gasdetektionsvorrichtung eine eigene Spannungsversorgungseinheit und ist damit unabhängig von einem stationären Spannungsversorgungsnetz. In einer Ausgestaltung lässt die erfindungsgemäße Gasdetektionsvorrichtung sich an der Kleidung eines Menschen befestigen oder in einer Hand tragen und warnt dann diesen Menschen vor brennbarem Zielgas. Bevorzugt gibt diese Gasdetektionsvorrichtung einen Alarm in einer von einem Menschen wahrnehmbaren Form aus, wenn ein brennbares Zielgas detektiert ist, beispielsweise visuell und / oder akustisch und / oder taktil, nämlich indem Vibrationen erzeugt werden. In einer Ausgestaltung übermittelt die Gasdetektionsvorrichtung einen Alarm an einen räumlich entfernten Empfänger, dass ein brennbares Zielgas detektiert ist, und / oder eine Nachricht, dass das Vorhandensein von brennbaren Zielgas ausgeschlossen werden kann.

Im Folgenden wird die Erfindung anhand eines Ausführungsbeispiels beschrieben. Hierbei zeigt
- Figur 1: eine aus dem Stand der Technik bekannte Gasdetektionsvorrichtung mit einem Detektor und einem Kompensator;
- Figur 2: eine Ausgestaltung der erfindungsgemäßen Gasdetektionsvorrichtung mit einem Detektor und einem Modulator;
- Figur 3: einen beispielhaften Aufbau eines als Pellistor ausgestalteten Detektors;
- Figur 4: eine beispielhafte Regelung der Modulator-Temperatur und der Detektor-Stromstärke;
- Figur 5: schematisch die Gasdetektionsvorrichtung von Figur 2 mit aktiviertem Modulator;
- Figur 6: schematisch die Gasdetektionsvorrichtung von Figur 2 mit deaktiviertem Modulator;
- Figur 7: den idealisierten zeitlichen Verlauf von Modulator-Spannung (oben) und Detektor-Temperatur (unten) beim Vorhandensein eines brennbaren Zielgases;
- Figur 8: den zeitlichen Verlauf von Figur 7 bei Abwesenheit eines brennbaren Zielgases;
- Figur 9: die zeitlichen Verläufe von Figur 7 bei sich verändernden Umgebungsbedingungen in der Detektorkammer, Zielgas vorhanden;
- Figur 10: die zeitlichen Verläufe in der Situation von Figur 9, kein Zielgas vorhanden;
- Figur 11: unterschiedliche idealisierte zeitliche Verläufe der Detektor-Temperatur bei verschiedenen Zielgasen;
- Figur 12: einen in einem Versuch gemessenen zeitlichen Verlauf der Detektor-Temperatur.

Im Ausführungsbeispiel wird die erfindungsgemäße Gasdetektionsvorrichtung eingesetzt, um ein vorgegebenes brennbares Gas, beispielsweise Methan (CH₄), zu detektieren. Dieses brennbare Gas wird im Folgenden als "Zielgas" bezeichnet. Selbstverständlich soll die Gasdetektionsvorrichtung keine Explosion und keinen Brand auslösen. Die Gasdetektionsvorrichtung des Ausführungsbeispiels ist bevorzugt als explosionsgeschütztes Gerät zugelassen und darf daher auch in einer explosionsgefährdeten Umgebung, beispielsweise in einem Bergwerk oder einer Raffinerie oder einer Lagerhalle, eingesetzt werden.

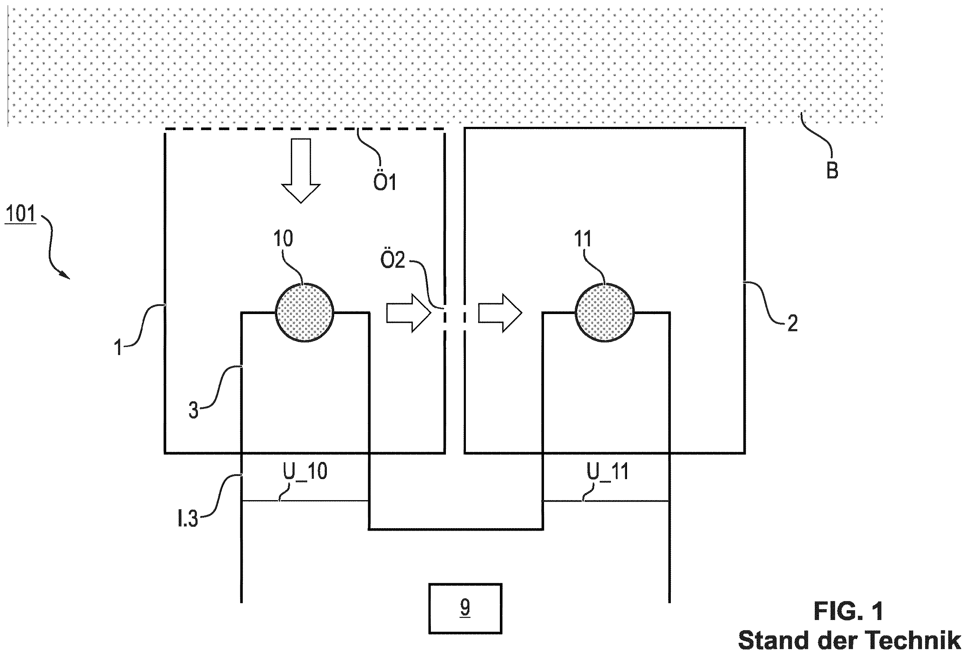

Figur 1 zeigt schematisch eine Gasdetektionsvorrichtung 101, die aus dem Stand der Technik her bekannt ist, beispielsweise aus DE 10 2017 005 713 A1, und sich als Wärmetönungssensor bezeichnen lässt. In einem zu überwachenden Bereich B kann ein brennbares Zielgas auftreten. Diese Gasdetektionsvorrichtung 101 umfasst
- eine Detektorkammer 1 mit einer äußeren Öffnung Ö1,
- eine Kompensatorkammer 2,
- eine innere Öffnung Ö2 zwischen der Detektorkammer 1 und der Kompensatorkammer 2,
- einen Detektor 10 in der Detektorkammer 1,
- einen Kompensator (Referenzeinheit) 11 in der Kompensatorkammer 2,
- eine elektrische Leitung 3, welche den Detektor 10 und den Kompensator 11 mit einer nicht gezeigten Stromquelle verbindet, und
- eine datenverarbeitende Auswerteeinheit 9.

Die Detektorkammer 1 steht über die äußere Öffnung Ö1 mit der Umgebung in einer Fluidverbindung. Die Kompensatorkammer 2 umgibt den Kompensator 11 vollständig, mit Ausnahme der inneren Öffnung Ö2. Ein Gas in dem Bereich B kann nur durch die innere Öffnung Ö2 in die Kompensatorkammer 2 fließen.

Ein nicht gezeigtes stabiles inneres Gehäuse umgibt die Detektorkammer 1 und die Kompensatorkammer 2. Alternativ umgibt jeweils ein inneres Gehäuse die Detektorkammer 1 und die Kompensatorkammer 2. die Stromquelle, die Auswerteeinheit 9 und weitere Bestandteile der Gasdetektionsvorrichtung 101 befinden sich außerhalb des oder jedes inneren Gehäuses und innerhalb eines äußeren Gehäuses.

Der Detektor 10 und der Kompensator 11 sind in Reihe geschaltet. Die Leitung 3, der Detektor 10 und der Kompensator 11 können Bestandteil einer Wheatstone'schen Messbrücke sein. Angedeutet werden weiterhin die Stromstärke I.3 in der Leitung 3, die Detektor-Spannung U_10, die am Detektor 10 anliegt, sowie die Kompensator-Spannung U_11, die am Kompensator 11 anliegt.

Der elektrische Strom, der durch die Leitung 3 fließt, bewirkt, dass der Detektor 10 und der Kompensator 11 sich erhitzen, und zwar abhängig von der Stromstärke I.3. Die Erhitzung des Detektors 10 bewirkt, dass ein zu detektierendes Zielgas in der Detektorkammer 1 oxidiert wird und dadurch Wärmeenergie freigesetzt wird. Dadurch erhitzt sich das Zielgas, und zwar abhängig von der Stromstärke 1.3. Diese Erhitzung des Zielgases induziert, dass der Detektor 10 sich weiter erhitzt. Die Temperatur des Detektors 10 steigt also an, und zwar abhängig von der Erhitzung des Zielgases. Ein in Figur 1 nicht gezeigter Sensor misst eine Detektions-Größe, beispielsweise die Detektor-Temperatur, die durch die Oxidation ansteigt, oder einen mit der Detektor-Temperatur korrelierenden Parameter.

Der durch die Leitung 3 fließende elektrische Strom bewirkt außerdem, dass der Kompensator 11 sich erhitzt. Der Kompensator 11 wird aber nicht von brennbarem Zielgas im Bereich B erhitzt. Veränderliche Umgebungsbedingungen, insbesondere eine veränderliche Umgebungstemperatur, wirken hingegen sowohl auf den Detektor 10 als auch auf den Kompensator 11. Ein nicht gezeigter Sensor misst die entsprechende Detektions-Größe des Kompensators 11.

Die datenverarbeitende Auswerteeinheit 9 erhält Messwerte von Sensoren, welche die Detektions-Größe für den Detektor 10 und die Detektions-Größe den Kompensators 11 messen. Diese Sensoren werden in Figur 1 nicht gezeigt. Eine signifikante Abweichung der gemessenen Werte für die Detektions-Größe zeigt das Vorhandensein des Zielgases. Das Fehlen einer signifikanten Abweichung zeigt, dass kein brennendes Zielgas in der Umgebung der Gasdetektionsvorrichtung 101 vorhanden ist - allgemeiner: dass die Konzentration von brennbarem Zielgas unterhalb einer Nachweisgrenze liegt.

In der in Figur 1 gezeigten Anordnung oxidiert der Detektor 10 einen großen Teil des Zielgases - falls ein solches Zielgas in der Detektorkammer 1 vorhanden ist. Das Gas, welches durch die Öffnung Ö2 in die Kompensatorkammer 2 gelangt, hat in einer Ausgestaltung eine deutlich geringere Konzentration an Zielgasen. Idealerweise ist es frei von Zielgas. In einer anderen Ausgestaltung vermag der Kompensator 11 es nicht, ein brennbares Zielgas zu oxidieren.

Sowohl der Detektor 10 als auch der Kompensator 11 sind bevorzugt als sogenannte Pellistoren aufgebaut. Auch für die Erfindung lassen sich Pellistoren verwenden.

Ein Pellistor umfasst kleine Pellets aus einem keramischen Material, das optional mit einem Katalysator durchsetzt ist. Bevorzugt umfasst der Pellistor einen Draht aus einem elektrisch leitenden Material, besonders bevorzugt aus Platin, der durch das keramischem Material hindurch geführt ist. Bevorzugt umfasst der Pellistor 10, 11 einen elektrisch leitenden Draht umfassend eine Spule mit Windungen, wobei der Draht durch das Innere der Keramik hindurch geführt ist und die Spule in die Keramik eingebettet ist. An den Draht im keramischen Material wird eine elektrische Spannung angelegt. Das keramische Material isoliert den Draht elektrisch und verhindert einen unerwünschten Kurzschluss.

Die Arbeitstemperatur des erhitzten Drahts allein reicht in vielen Fällen nicht aus, um ein Zielgas zu oxidieren. Das katalytische Material in oder auf der Keramik bewirkt, dass ein brennbares Gas oxidiert wird, und trägt in vielen Fällen dazu bei, dass ein brennbares Zielgas katalytisch oxidiert und nicht auf eine andere Weise oxidiert, insbesondere dazu, dass es nicht verbrennt. Ein Pellistor ohne katalytisches Material vermag es in vielen Fällen nicht oder nur bei einer unerwünscht hohen Arbeitstemperatur, ein brennbares Zielgas zu oxidieren.

Der elektrische Widerstand eines Pellistors verändert sich abhängig von seiner eigenen Temperatur und damit auch abhängig von der Temperatur des umgebenden Zielgases und der umgebenden Luft, auch wenn diese frei von Zielgas ist. In der Regel steigt der elektrische Widerstand mit steigender Temperatur, wobei häufig ein linearer Zusammenhang angenommen werden kann. Andererseits verändert sich die Temperatur des Pellistors abhängig von der anliegenden elektrischen Spannung, welche eine Erhitzung des Drahts bewirkt.

In einer Ausgestaltung von Figur 1 ist in die Keramik des Detektors 10 ein katalytisches Material eingelassen, in die Keramik des Kompensator 11 hingegen nicht. Der Strom, der in der Leitung 3 mit der Stromstärke I fließt, erhitzt die in Reihe geschalteten Pellistoren 10 (Detektor) und 11 (Kompensator) auf eine Arbeitstemperatur, die beispielsweise zwischen 400 °C und 500 °C liegt. Sowohl der Detektor 10 als auch der Kompensator 11 erhitzen sich durch den elektrischen Strom. Aufgrund des katalytischen Materials vermag der Detektor 10 das brennbare Zielgas in der Detektorkammer 1 oxidieren. Die Wärmeenergie, die beim Oxidieren des Zielgases freigesetzt wird, verändert die Temperatur des Detektors 10. Der Kompensator 11 verändert seine Temperatur hingegen nur aufgrund von veränderten Umgebungsbedingungen. Die Temperatur der Umgebungsluft beeinflusst die Temperatur beider Pellistoren 10 und 11. Diese Temperatur-Änderung bewirkt, dass sich der jeweilige elektrische Widerstand der Pellistoren 10 und 11 verändert. Diese Veränderung des elektrischen Widerstands bewirkt eine Veränderung der am Pellistor 10 bzw. 11 anliegenden elektrischen Spannung und / oder der Stromstärke. Sowohl der elektrische Widerstand als auch die elektrische Spannung und Stromstärke korrelieren also mit dem Vorhandensein eines brennbaren Zielgases. In einer Ausgestaltung wird die Differenz aus der Temperatur des Detektors 10 und der Temperatur des Kompensators 11 gebildet. Diese Temperaturdifferenz ist ein Maß für die Konzentration von brennbarem Zielgas.

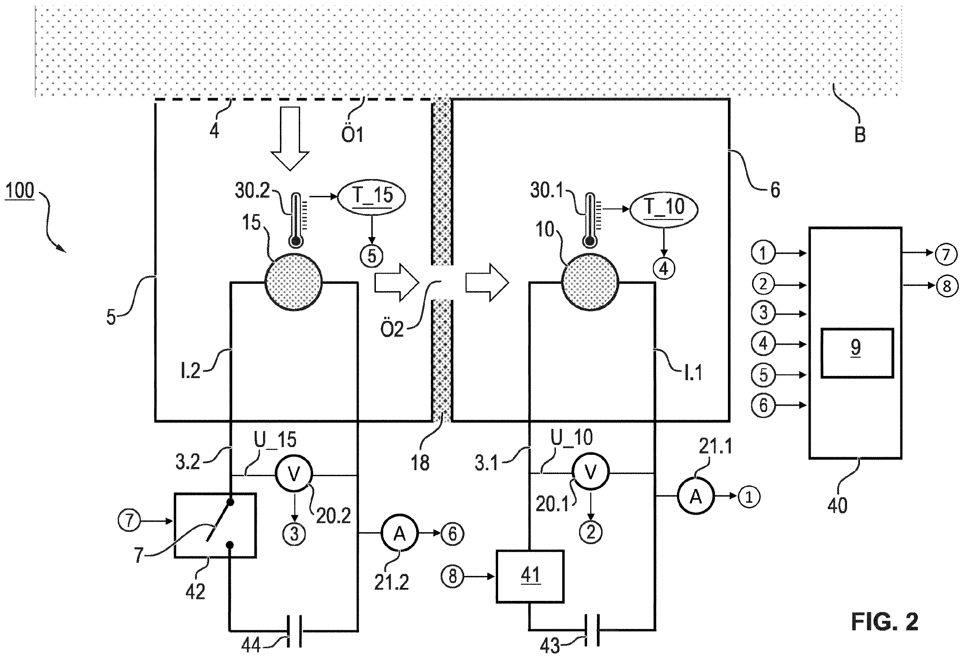

Figur 2 zeigt den prinzipiellen Aufbau einer Ausgestaltung der erfindungsgemäßen Gasdetektionsvorrichtung 100. Gleiche Bestandteile haben die gleichen Bezugszeichen wie in Figur 1.

Die Gasdetektionsvorrichtung 100 gemäß Figur 2 besitzt ebenfalls zwei Kammern, nämlich eine Modulatorkammer 5 und eine Detektorkammer 6. Die Modulatorkammer 5 kann genauso aufgebaut sein wie die Detektorkammer 1 von Figur 1, die Detektorkammer 6 genauso wie die Kompensatorkammer 2. Die Modulatorkammer 5 und die Detektorkammer 1 sind von einem einzigen stabilen inneren Gehäuse oder jeweils einem stabilen inneren Gehäuse umgeben, welches in Figur 2 nicht gezeigt wird. Die Gasdetektionsvorrichtung 100 umfasst ebenfalls ein äußeres Gehäuse.

Die Modulatorkammer 5 steht über die äußere Öffnung Ö1 mit dem zu überwachenden Bereich B in einer äußeren Fluidverbindung. In einer Ausgestaltung nimmt die äußere Öffnung Ö1 die gesamte Fläche einer Seite der Modulatorkammer 5 ein. Bis auf die äußere Öffnung Ö1 umgibt die Modulatorkammer 5 vollständig und gasdicht den Modulator 15.

In Figur 2 ist die äußere Öffnung Ö1 oben an der Modulatorkammer 5 angeordnet. Bevorzugt schützt ein mechanisches Gitter die Modulatorkammer 5 vor mechanischen Einflüssen. In einer anderen Ausgestaltung zeigt die äußere Öffnung Ö1 hingegen senkrecht oder schräg nach unten, damit die Gefahr verringert ist, dass Regen oder Staubpartikel oder sonstige starre Gegenstände in die Modulatorkammer 5 gelangen können. Regen und starre Gegenstände können daher die Messergebnisse nicht wesentlich verfälschen.

Bevorzugt verhindert ein Explosionsschutzgitter 4 aus einem Drahtgewebe oder einem Filtermaterial, beispielsweise aus einem Sintermaterial, dass Flammen durch die äußere Öffnung Ö1 hindurch aus der Modulatorkammer 5 austreten. Ein gasdurchlässiger Partikelfilter verhindert in einer Ausgestaltung, dass Partikel in die Modulatorkammer 5 gelangen. In einer bevorzugten Ausgestaltung kann jedes brennbare Zielgas in die Modulatorkammer 5 und durch die Öffnung Ö2 hindurch in die Detektorkammer 6 gelangen, weil kein Gasfilter dieses Zielgas herausfiltert.

Die Detektorkammer 6 steht über eine innere Öffnung Ö2 in einer inneren Fluidverbindung mit der Modulatorkammer 5. Bis auf die innere Öffnung Ö2 ist die Detektorkammer 6 des Ausführungsbeispiels gasdicht gegen die Umgebung abgedichtet. Gas aus dem Bereich B kann daher nur durch die äußere Öffnung Ö1, die Modulatorkammer 5 und die innere Öffnung Ö2 hindurch in die Detektorkammer 6 fließen, aber nicht direkt aus dem Bereich B in die Detektorkammer 6.

Bevorzugt befindet sich zwischen der Modulatorkammer 5 und der Detektorkammer 6 eine thermische Barriere 18, die in Figur 2 schematisch angedeutet ist. Dank der thermischen Barriere 18 ist die Detektorkammer 6 bis zu einem gewissen Grade von der Modulatorkammer 5 thermisch isoliert. Dank der bewirkten thermischen Isolierung beeinflusst die Temperatur des Modulators 15 nur in relativ geringem Umfang die Temperatur des Detektors 10.

Im Ausführungsbeispiel sind der Detektor 10 und der Modulator 15 bevorzugt ebenfalls als Pellistoren aufgebaut. Sie können insbesondere so ausgestaltet sein, wie dies für den Detektor 10 mit Bezug auf Figur 1 beschrieben wurde. Auch der Modulator 15 weist katalytisches Material in der Keramikschicht auf und vermag Zielgas zu oxidieren und beim Oxidieren zu verbrennen. Die Erfindung erspart also die Notwendigkeit, zwei unterschiedliche Pellistoren bereitstellen zu müssen, nämlich einen Pellistor mit katalytischem Material und einen Pellistor ohne katalytisches Material in der Keramikschicht.

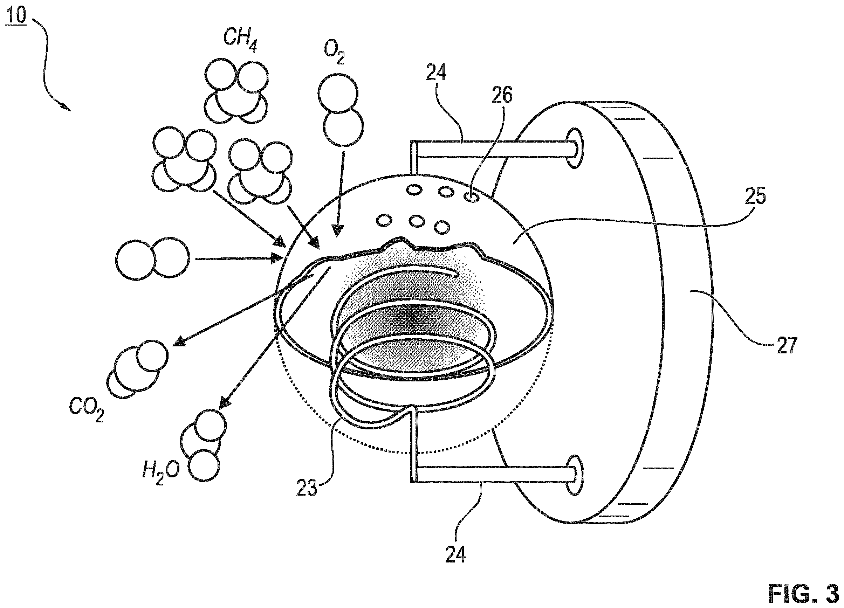

Figur 3 zeigt beispielhaft den Aufbau des Detektors 10, der als Pellistor ausgestaltet ist. Der Modulator 15 kann gleichartig aufgebaut sein, braucht aber nicht notwendigerweise dieselben elektrischen und thermischen Eigenschaften aufzuweisen wie der Detektor 10. Der Detektor 10 gemäß Figur 3 umfasst einen Draht aus einem elektrisch leitenden Material, bevorzugt aus Platin. Dieser Draht umfasst ein spiralförmiges heizendes Segment 23, also eine Spule, sowie zwei elektrische Verbindungen 24 für dieses heizende Segment 23. Zwei mechanische Halterungen halten die beiden elektrischen Verbindungen 24. Der Detektor 10 umfasst weiterhin
- eine elektrische Isolierung in Form einer Ummantelung 25 um das heizende Segment 23, die aus einem keramischen Material hergestellt ist und den das heizende Segment 23 vollständig umgibt und idealerweise in flächigem Kontakt mit der gesamten Länge des heizendes Segments 23 steht Beschichtung der äußeren Oberfläche der keramischen Ummantelung 25, welche aus einem katalytischen Material besteht und durch Kreise 26 angedeutet ist, und
- eine Montageplatte 27.

Die elektrische Isolierung 25 verhindert, dass die Spule 23 in sich kurzgeschlossen wird, und stellt eine ausreichende mechanische Stabilität sicher. Die elektrische Isolierung 25 stellt einen thermischen Kontakt zwischen dem heizenden Segment 23 und der Beschichtung 26 her. Einerseits bewirkt das stromdurchflossene und erhitzte heizende Segment 23 dank des thermischen Kontakts, dass ein Zielgas oxidiert wird. Andererseits wirkt die Wärmeenergie, die bei der Oxidation freigesetzt wird, dank des thermischen Kontakts auf das heizende Segment 23 und erhitzt dieses weiter.

Die Beschichtung 26 aus dem katalytischen Material ist bevorzugt so ausgestaltet, dass der kugelförmige Detektor 10 eine poröse Oberfläche aufweist. Dadurch ist die Oberfläche des Detektors 10 größer verglichen mit einer glatten Oberfläche. Außerdem dringt ein Gasgemisch in tieferliegende Schichten der Ummantelung 25 ein. Die vergrößerte Oberfläche und das Eindringen des Gasgemisches verbessern die Oxidation des brennenden Zielgases. Auch beim Oxidieren des Zielgases wird die poröse Struktur der Detektor-Oberfläche aufrechterhalten, was durch die elektrischen Isolierung des heizenden Segments 23 aus dem keramischen Material bewirkt wird. Bevorzugt hat die Ummantelung 25 die Form einer Vollkugel, wobei sich im Inneren dieser Vollkugel einzelne Blasen befinden können, die beim Herstellen erzeugt worden sind.

Wie in Figur 3 angedeutet ist, bewirkt der Detektor 10 eine Oxidation des Zielgases, hier beispielhaft Methan (CH₄). Hierbei werden CH₄ und O₂ in H2o und CO₂ umgewandelt. Bei dieser Oxidation wird Wärmeenergie freigesetzt.

Möglich, aber dank der Erfindung nicht zwingend erforderlich ist, dass die beiden Pellistoren 10, 15 ihre elektrischen Eigenschaften in gleicher Weise abhängig von der Temperatur verändern. Der Verzicht auf diese Anforderung, die in der Praxis oft schwer einzuhalten ist, resultiert aus einer anderen Wirkungsweise, die nachfolgend beschrieben wird. Der Detektor 10 ist in einer Ausführungsform so aufgebaut wie das Messelement 130 oder 330 von DE 10 2017 011 530 A1.

Die elektrische Leitung 3.1 von Figur 2 verbindet den Detektor 10 mit einer schematisch gezeigten Spannungsquelle 43. Die elektrische Leitung 3.2 verbindet den Modulator 15 mit einer schematisch gezeigten Spannungsquelle 44. Diese Spannungsquellen 43, 44 können von einem stationären Spannungsversorgungsnetz bereitgestellt werden. Möglich ist auch, dass die Spannungsquelle 43, 44 jeweils eine lokale Spannungsversorgungseinheit sind, beispielsweise eine Batterie oder ein Akkumulator.

Der Modulator 15 und der Detektor 10 sind im Ausführungsbeispiel elektrisch dergestalt geschaltet, dass sich unabhängig voneinander jeweils eine elektrische Spannung an diese anlegen lässt. Der Modulator 15 und der Detektor 10 sind also nicht in Reihe geschaltet. Im Ausführungsbeispiel lässt sich daher die Spannung U_15, welche an dem Modulator 15 anliegt, unabhängig von der Spannung U_10, welche an dem Detektor 10 anliegt, verändern.

Eine schematisch gezeigte Spannungsveränderungseinheit 42 vermag die Spannung U zu verändern, die am Modulator 15 anliegt. Bevorzugt lässt diese Spannungsveränderungseinheit 42 sich von außen ansteuern und verändert die Spannung U_15 so, dass diese idealerweise einem vorgegebenen Verlauf folgt und hierbei oszilliert. Bevorzugt wird die elektrische Spannung U_15 gepulst angelegt. In einer simplen Realisierungsform dieses Pulsens vermag ein von außen ansteuerbarer Schalter 7 der Spannungsveränderungseinheit 42 die elektrische Leitung 3.2 wahlweise zu unterbrechen oder freizuschalten. Je nach Stellung des Schalters 7 wird der Modulator 15 mit Strom versorgt oder nicht mit Strom versorgt.

In der gezeigten simplen Realisierungsform bleibt die Spannung U_15, welche am Modulator 15 anliegt, zeitlich konstant. In einer allgemeineren Realisierungsform lässt sich ein Spannungsregler der Spannungsveränderungseinheit 42 von außen ansteuern, um die anliegende Spannung U_15 zu verändern.

Eine Spannungsveränderungseinheit 41 vermag die elektrische Spannung U_10, die am Detektor 10 anliegt, zu verändern.

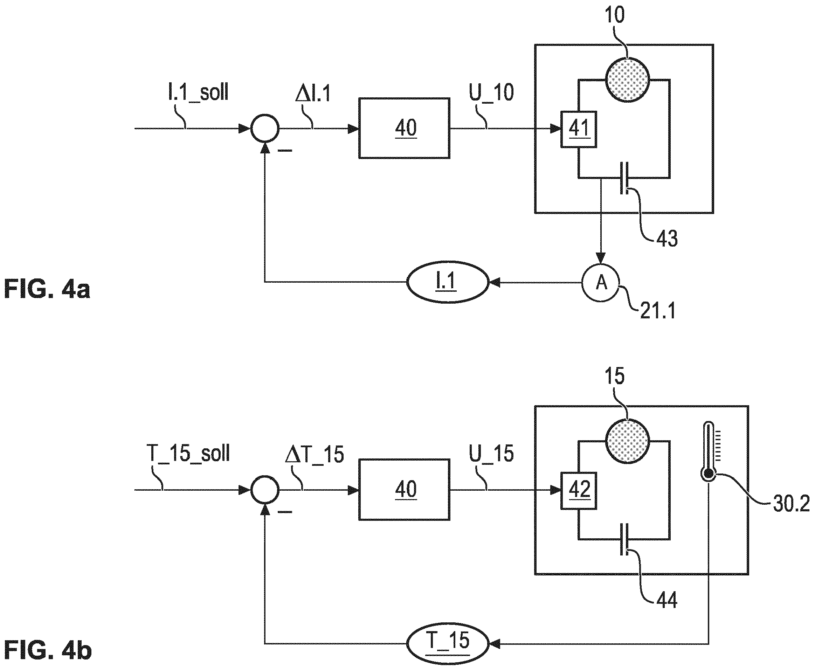

Möglich ist, dass die elektrische Spannung U_15, welche am Modulator 15 anliegt, oszilliert, insbesondere einen rechteckigen Verlauf aufweist, also zwischen zwei Werten hin- und herspringt. Auch die Spannung U_10, welche am Detektor 10 anliegt, ist in einer Ausgestaltung zeitlich veränderbar, und zwar mithilfe der Spannungsveränderungseinheit 41. Zwei weitere abweichende bevorzugte Ausführungsformen werden weiter unten mit Bezug auf Figur 4 erläutert.

In der beispielhaften Ausgestaltung gemäß Figur 2 umfasst die erfindungsgemäße Gasdetektionsvorrichtung 100 die folgenden Sensoren:
- einen Spannungs-Sensor 20.1, der die aktuell am Detektor 10 anliegende elektrische Spannung U_10 misst,
- einen Spannungs-Sensor 20.2, der die aktuell am Modulator 15 anliegende elektrische Spannung U_15 misst,
- einen Stromstärken-Sensor 21.1, der die aktuelle Stärke I.1 des durch den Detektor 10 fließenden elektrischen Stroms misst,
- einen Stromstärken-Sensor 21.2, der die aktuelle Stärke I.2 des durch den Modulator 15 fließenden elektrischen Stroms misst,
- optional einen Temperatur-Sensor 30.1, der ein Maß für die aktuelle Temperatur T_10 des Detektors 10 misst, beispielsweise direkt die Temperatur, und
- optional einen Temperatur-Sensor 30.2, der ein Maß für die aktuelle Temperatur T_15 des Modulators 15 misst, beispielsweise direkt die Temperatur.

In einer bevorzugten Ausgestaltung empfängt die Auswerteeinheit 9 Messwerte von dem Spannungs-Sensor 20.1 und dem Stromstärken Sensor 21.1 und berechnet aus der Spannung U_10 und der Stromstärke 1.1 des Detektors 10 den aktuellen Wert des elektrischen Widerstands des Detektors 10. Bekanntlich korreliert in vielen Situationen die Temperatur eines von Strom durchflossenen metallischen Bauteils mit seinem elektrischem Widerstand dergestalt, dass der elektrische Widerstand umso größer ist, je höher die Temperatur ist. In vielen Fällen sind diejenigen Parameter des Detektors 10, welche die Abhängigkeit zwischen der Temperatur und dem elektrischen Widerstand beeinflussen, aufgrund der Konstruktion des Detektors 10 ausreichend genau bekannt. Gemäß einer bevorzugten Ausgestaltung berechnet die Auswerteeinheit 9 die Temperatur T_10 des Detektors 10 aus der Spannung U_10 und der Stromstärke 1.1. In dieser Ausgestaltung fungiert die Temperatur T_10 des Detektors 10 als die Detektions-Größe, welche von der Oxidation eines Zielgases in der Detektorkammer 6 beeinflusst wird.

In einer anderen Ausgestaltung fungiert die am Detektor 10 anliegende Spannung U_10 als die Detektions-Größe. Falls die Stromstärke 1.1 des durch den Detektor 10 fließenden Stroms bekannt ist, so ist die anliegende Spannung U_10 ein Maß für den elektrischen Widerstand und damit ein Maß für die Temperatur T_10 des Detektors 10 und damit auch ein Maß für die Wärmeenergie, die bei der Oxidation eines brennbaren Zielgases freigesetzt wird. Diese Ausgestaltung erspart die Notwendigkeit, die Temperatur T_10 direkt zu messen oder zu berechnen, spart also einen Temperatur-Sensor 30.1 ein.

Entsprechend empfängt die Auswerteeinheit 9 Messwerte von dem Spannungs-Sensor 20.2 und dem Stromstärken-Sensor 21.2, berechnet den aktuellen Wert des elektrischen Widerstands des Modulators 15 und leitet aus diesem die aktuelle Temperatur T_15 des Modulators 15 ab.

Bevorzugt steht die Auswerteeinheit 9 in einer Datenverbindung mit einem Empfänger, beispielsweise mit einer räumlich entfernten Zentrale. Falls die Auswerteeinheit 9 ein brennbares Zielgas detektiert hat, so löst sie den Schritt aus, dass eine Nachricht an den Empfänger übermittelt wird und eine Ausgabeeinheit des Empfängers einen Alarm in einer von einem Menschen wahrnehmbaren Form ausgibt. In einer anderen Ausgestaltung umfasst die Gasdetektionsvorrichtung 100 eine Alarmeinheit, welche dann einen Alarm in einer von einem Menschen wahrnehmbaren Form ausgibt, wenn ein brennbares Zielgas detektiert ist.

Die Gasdetektionsvorrichtung 100 von Figur 2 umfasst weiterhin ein datenverarbeitendes Steuergerät 40, welches die Auswerteeinheit 9 umfasst. Bevorzugt führt dieses Steuergerät 40 eine automatische Regelung für den Detektor 10 und eine automatische Regelung für den Modulator 15 durch. Eine regelbare Größe des Detektors 10 bzw. des Modulators 15 ist die jeweilige Regelgröße dieser Regelung. Die regelbare Größe des Detektors 10 beeinflusst die Detektions-Größe, insbesondere die Temperatur T_10 oder die elektrische Spannung U_10. Die regelbare Größe des Modulators 15 beeinflusst den Vorgang, dass der Modulator 15 brennbares Zielgas oxidiert.

Jeweils ein gewünschter oder geforderter zeitlicher Verlauf dieser Regelgröße wird vorgegeben und fungiert als die Führungsgröße. Das Regelungsziel ist, dass der tatsächliche zeitliche Verlauf der Regelgröße, also der regelbaren Größe, dem vorgegebenen zeitlichen Verlauf folgt. Die Stellgröße dieser Regelung ist im gezeigten Beispiel die am Detektor 10 anliegende Spannung U_10 bzw. die am Modulator 15 anliegende Spannung U_15. Das Steuergerät 40 steuert die beiden Spannungsveränderungseinheiten 41 und 42 an, um die Spannung U10 bzw. U_15 zu verändern und dadurch zu bewirken, dass die jeweilige Regelgröße der vorgegebenen Führungsgröße folgt.

Möglich ist, dass für den Detektor 10 eine erste Führungsgröße und damit eine erste Regelgröße verwendet wird und für den Modulator 15 eine zweite Führungsgröße und damit eine zweite Regelgröße.

In einer Ausgestaltung wird die tatsächliche Temperatur T_15 des Modulators 15 als Regelgröße für den Modulator 15 verwendet. Diese tatsächliche Temperatur T_15 wird direkt gemessen, beispielsweise durch den Temperatur-Sensor 30.2, oder indirekt, indem aus der Spannung U_15 und der Stromstärke I.2 der elektrische Widerstand des Modulators 15 abgeleitet wird und aus dem elektrischen Widerstand die Temperatur T_15.

Die Gasdetektionsvorrichtung 100 wendet einen vorgegebenen funktionalen Zusammenhang, insbesondere eine Kennlinie, zwischen der Temperatur und dem elektrischen Widerstand an. In einer Ausgestaltung ist dieser funktionale Zusammenhang vorgegeben und in einem Datenspeicher der Gasdetektionsvorrichtung 100 abgespeichert. In einer anderen Ausgestaltung wird bei einer vorhergehenden Justierung eine vorgegebene Temperatur des Modulators 15 eingestellt, und der elektrische Widerstand, den der Modulator 15 bei dieser Temperatur aufweist, wird gemessen. Diese Justierung liefert einen Referenzpunkt der Kennlinie. Bei Bedarf wird diese Justierung erneut durchgeführt. Die Gasdetektionsvorrichtung 100 verwendet diesen Referenzpunkt sowie eine abgespeicherte rechnerauswertbare Berechnungsvorschrift, um für andere Werte des elektrischen Widerstands die jeweilige Temperatur zu ermitteln.

Die vorgegebene Soll-Temperatur T_15_soll des Modulators 15 oszilliert und weist beispielsweise einen rechteckigen Verlauf auf, d.h. die Temperatur springt zwischen zwei verschiedenen Werten hin- und her. Bei dem höheren Temperatur-Wert oxidiert der Modulator 15 eine größere Menge des Zielgases in der Modulatorkammer 5, vorausgesetzt brennbares Zielgas ist in der Modulatorkammer 5 vorhanden.

Als Regelgröße für den Detektor 10 wird in einer Ausgestaltung die Stromstärke 1.1 des Stroms verwendet, der durch den Detektor 10 fließt. Weil die Temperatur des Gases in der Detektorkammer 6 und dadurch auch die Temperatur T_10 des Detektors 10 zeitlich veränderlich sein können und weil die Temperatur T_10 des Detektors 10 dessen elektrischen Widerstand beeinflusst, wird die am Detektor 10 anliegende Spannung U_10 mit dem Ziel geregelt, dass die Stromstärke 1.1 einem vorgegebenen zeitlichen Verlauf I.1_soll folgt, insbesondere dass sie konstant bleibt.

Figur 4a) zeigt beispielhaft, wie das Steuergerät 40 die Stromstärke 1.1 des Stroms 1.1, der durch den Detektor 10 fließt, regelt. Figur 4b) zeigt beispielhaft, wie das Steuergerät 40 die Temperatur T_15 des Modulators 15 regelt. In diesen schematischen Darstellungen haben die Bezugszeichen die gleichen Bedeutungen wie in Figur 2. Außerdem bezeichnen
- 1.1 _soll einen vorgegebenen zeitlichen Verlauf der gewünschten oder geforderten Stromstärke 1.1, insbesondere einen vorgegebenen Wert,
- ΔI.1 die Regelabweichung zwischen der gewünschten Stromstärke I.1_soll und der gemessenen Stromstärke 1.1,
- T_15_soll einen vorgegebenen zeitlichen Verlauf der gewünschten oder geforderten Modulator-Temperatur T_15 und
- ΔT_15 die Regelabweichung zwischen der gewünschten Modulator-Temperatur T_15_soll und der gemessenen Modulator-Temperatur T_15.

In dem gezeigten Beispiel werden die Stromstärke 1.1 durch den Detektor 10 sowie die Temperatur T_15 des Modulators 15 geregelt. Möglich ist auch, folgende Größe für den Detektor 10 und/oder für den Modulator 15 zu regeln, wobei für den Detektor 10 und den Modulator 15 dieselbe Größe oder unterschiedliche Größen geregelt werden können:
- die Stärke I.1, I.2 des Stroms, der durch den Detektor 10 bzw. den Modulator 15 fließt,
- die Spannung U_10, U_15, die am Detektor 10 bzw. am Modulator 15 anliegt,
- der elektrische Widerstand des Detektors 10 bzw. des Modulators 15, welcher bekanntlich von der Temperatur T_10 bzw. T_15 abhängt,
- direkt die Temperatur T_10, T_15 des Detektors 10 bzw. des Modulators 15,
- die vom Detektor 10 bzw. vom Modulator 15 aufgenommene elektrische Leistung, welche bekanntlich vom elektrischen Widerstand und damit von der Temperatur T_10 bzw. T_15 abhängt.

Wie bereits erwähnt, vermag der Detektor 10 ein brennbares Zielgas zu oxidieren, welches sich in der Detektorkammer 6 befindet. Das Oxidieren des Zielgases verändert die Temperatur in der Detektorkammer 6 und auch die Temperatur T_10 des Detektors 10. Diese Temperatur T_10 wird, so wie oben beschrieben, direkt oder indirekt gemessen. Je größer die Konzentration des Zielgases in der Detektorkammer 6 ist, desto stärker wird der Detektor 10 erhitzt. Die gemessene Temperatur T_10 des Detektors 10 korreliert daher mit der gesuchten Konzentration, insbesondere mit dem Vorhandensein oder Nicht-Vorhandensein - des brennbaren Zielgases in der Detektorkammer 6.

In einer möglichen Implementierung der gezeigten Ausgestaltung misst der Temperatur-Sensor 30.1 direkt die Temperatur T_10 des Detektors 10. Die gemessene Temperatur T_10 wird an die Auswerteeinheit 9 übermittelt. Möglich ist, dass zusätzlich die gemessene Spannung U_10, die am Detektor 10 anliegt, sowie die gemessene Stromstärke 1.1 des durch den Detektor 10 fließenden Stroms gemessen werden. Aus der Spannung U_10 und der Stromstärke 1.1 wird der aktuelle elektrische Widerstand des Detektors 10 hergeleitet. Der elektrische Widerstand des Detektors 10 ist zeitlich veränderlich und korreliert mit dessen Temperatur T_10. Die beiden gerade beschriebenen Ausgestaltungen, nämlich einerseits direkt die Temperatur T_10 zu messen und andererseits den elektrischen Widerstand und aus dem elektrischen Widerstand die Temperatur T_10 herzuleiten, lassen sich kombinieren. Diese Kombination vergrößert die Zuverlässigkeit der Detektion und ermöglicht es oft, eine Gasdetektionsvorrichtung mit Redundanz bereitzustellen. Möglich ist auch, die elektrische Spannung U_10 als Detektions-Größe zu verwenden.

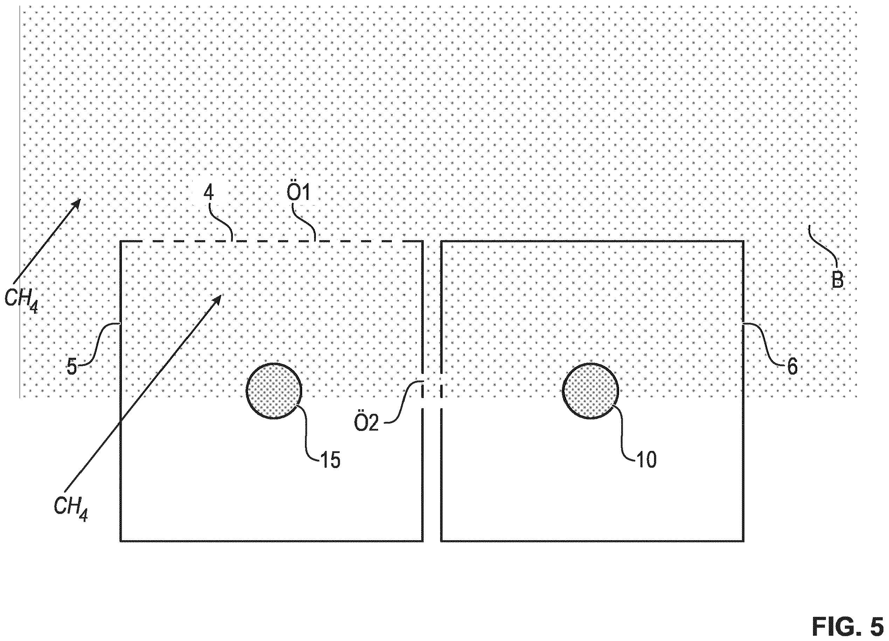

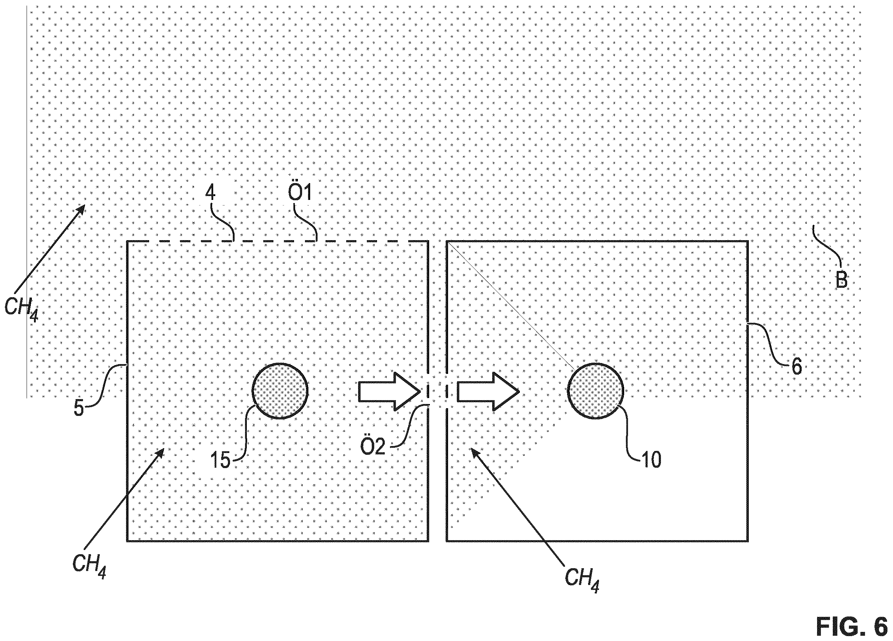

Figur 5 und Figur 6 zeigen schematisch die Gasdetektionsvorrichtung 100 von Figur 2, wobei ein brennbares Zielgas, hier Methan (CH₄), in dem zu überwachenden Bereich B vorhanden ist. Das Zielgas fließt durch die äußere Öffnung Ö1 hindurch in die Modulatorkammer 5. In Figur 5 und Figur 6 ist das Zielgas CH₄ durch Punkte angedeutet.

Figur 5 zeigt eine Situation, in welcher der Modulator 15 auf eine hohe Temperatur erhitzt ist. Der als Pellistor realisierte Modulator 15 oxidiert den größten Teil des Zielgases in der Modulatorkammer 5 und verbrennt damit das meiste Zielgas. Daher fließt praktisch kein Zielgas durch die innere Öffnung Ö2 hindurch in die Detektorkammer 6.

Figur 6 zeigt eine Situation, in welcher der Modulator 15 auf eine niedrigere Temperatur erhitzt oder sogar ausgeschaltet ist. Der Modulator 15 oxidiert nur einen geringen Teil des Zielgases oder überhaupt kein Zielgas. Daher fließt wenigstens ein Teil des brennbaren Zielgases durch die innere Öffnung Ö2 hindurch in die Detektorkammer 6. Der als Pellistor realisierte Detektor 10 oxidiert und verbrennt daher das Zielgas in der Detektorkammer 6 und wird weiter erhitzt.

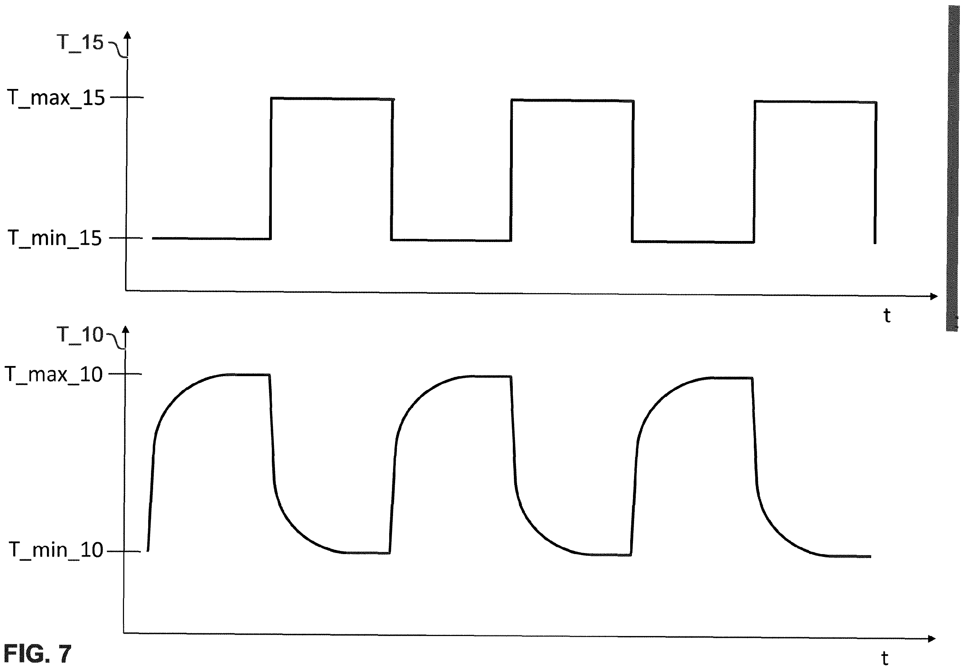

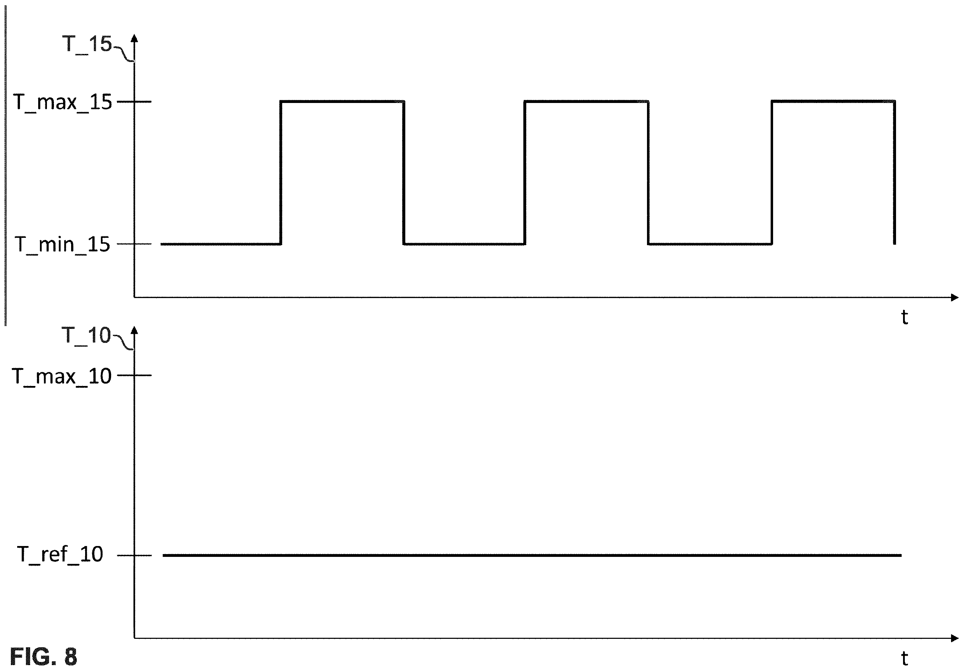

Figur 7 und Figur 8 zeigen beispielhaft idealisierte zeitlichen Verläufe der Temperatur T_15 des Modulator 15 (oben) und die Temperatur T_10 des Detektors 10 (unten). Die Modulator-Temperatur T_15 wird bevorzugt so geregelt, wie dies gerade mit Bezug auf Figur 4b) beschrieben wurde. Die Detektor-Temperatur T_10 wird so wie oben beschrieben direkt oder indirekt gemessen. In dem gezeigten Beispiel wird die Modulator-Temperatur T_15 so geregelt, dass sie zwischen der niedrigeren Temperatur T_min_15 und der höheren Temperatur T_max_15 hin und her schwankt, in dem gezeigten idealisierten Beispiel ohne Zeitverzug. Bei der höheren Temperatur T_max_15 oxidiert der Modulator 15 mehr brennbares Zielgas in der Modulatorkammer 5 als bei der niedrigeren Temperatur T_min_15. Die zeitlichen Verläufe der Modulator-Temperatur T_15 stimmen in Figur 7 und Figur 8 überein.

Die am Detektor 10 anliegende elektrische Spannung U_10 wird in diesem Beispiel so geregelt, dass die Stärke 1.1 des durch den Detektor 10 fließenden Stroms konstant bleibt. Wie diese Regelung durchgeführt wird, wurde mit Bezug auf Figur 4a) beschrieben. Der Spannungs-Sensor 20.1 misst die Spannung U_10, die am Detektor 10 anliegt und die in einer Ausgestaltung als Detektions-Größe fungiert. Die Wärmeenergie, welche der elektrische Strom dem Detektor 10 zuführt, hängt von den bekannten Größen 1.1 und U_10 ab.

Falls in dem überwachten Bereich B kein brennbares Zielgas vorhanden ist, so verändert sich die Detektor-Temperatur T_10 nur relativ langsam, nämlich aufgrund von sich verändernden Umgebungsbedingungen. Bei der Oxidation eines Zielgases verändert sich die Detektor-Temperatur T_10 hingegen viel rascher. Sich verändernde Umgebungsbedingungen bewirken in aller Regel keine rasche Oszillation der Detektor-Temperatur T_10. Zeitliche Schwankungen der Detektor-Temperatur T_10 resultieren daher aus einer zeitlich variierenden Konzentration des Zielgases in der Detektorkammer 6.

In dem in Figur 2 gezeigten Beispiel wird die Detektor-Temperatur T_10 gemessen und als Detektions-Größe verwendet. Der elektrische Widerstand des Detektors 10 korreliert mit dessen Temperatur T_10, und die Detektor-Temperatur T_10 korreliert mit der Konzentration des Zielgases in der Detektorkammer 6. Falls die Stromstärke 1.1 und die am Detektor 10 anliegende Spannung U_10 gemessen werden, lässt sich der elektrische Widerstand herleiten und ist ein Maß für die Detektor-Temperatur T_10. Falls insbesondere die Stromstärke 1.1 konstant gehalten wird (eine Ausgestaltung der Regelung gemäß Figur 4 a), so lässt sich die am Detektor 10 anliegende elektrische Spannung U_10 als Detektions-Größe verwenden. Falls umgekehrt die Spannung U_10 konstant gehalten wird, lässt sich die Stromstärke 1.1 als Detektions-Größe verwenden.

Figur 7 und Figur 8 zeigen idealisiert die zeitlichen Verläufe der Temperaturen T_15 und T_10 in einer Situation, in der eine Menge eines Gasgemisches aus dem überwachten Bereich B in die Modulatorkammer 5 fließt. Ein Teil dieses Gasgemisches fließt aus der Modulatorkammer 5 durch die innere Öffnung Ö2 hindurch in die Detektorkammer 6. In der in Figur 7 gezeigten Situation ist in dem zu überwachenden Bereich B ein brennbares Zielgas vorhanden, in der in Figur 8 gezeigten Situation nicht. Der zeitliche Verlauf der Modulator-Temperatur T_15 stimmt in Figur 7 und Figur 8 überein.

In einem Zeitintervall, in dem die Modulator-Temperatur T_15 den höheren Wert T_max_15 annimmt, wird ein erheblicher Teil des brennbaren Zielgases in der Modulatorkammer 5 oxidiert - natürlich nur, wenn das Gasgemisch ein brennbares Zielgas enthält. Daher enthält das Gasgemisch, das durch die innere Öffnung Ö2 in die Detektorkammer 6 fließt, auch dann nur eine relativ geringe Konzentration des brennbaren Zielgases, wenn der Bereich B dieses Zielgas enthält. Der erhitzte Detektor 10 vermag daher nur wenig oder sogar überhaupt kein Zielgas in der Detektorkammer 6 zu oxidieren und erhitzt sich daher auch nur wenig durch brennbares Zielgas.

In einem Zeitintervall, in dem die Modulator-Temperatur T_15 den niedrigeren Wert T_min_15 annimmt, wird nur ein geringer oder gar kein Teil des brennbaren Zielgases in der Modulatorkammer 6 oxidiert. Daher enthält das Gasgemisch, das durch die innere Öffnung Ö2 in die Detektorkammer 6 fließt, relativ viel brennbares Zielgas - vorausgesetzt, dass das Gasgemisch dieses brennbare Zielgas enthält.

Figur 7 zeigt, dass der zeitliche Verlauf der Detektor-Temperatur T_10 zwischen einem Maximalwert T_max_10 und einen Minimalwert T_min_10 oszilliert, vorausgesetzt der Bereich B enthält das brennbare Zielgas. In einem Zeitintervall, in dem der Modulator 15 die niedrigere Temperatur T_min_15 aufweist, steigt die Detektor-Temperatur T_10 bis zum Maximalwert T_max_10 an. In einem Zeitintervall, in dem der Modulator 15 die höhere Temperatur T_max_15 aufweist, fällt die Detektor-Temperatur T_10 wieder bis zum Minimalwert T_min_10 ab.

Figur 8 zeigt in eine idealisierten Darstellung die zeitlichen Verläufe der Modulator-Temperatur T_15 und der Detektor-Temperatur T_10, wobei die elektrischen Spannungen U_15 und U_10 dieselben Verläufe aufweisen, die zu den Verläufen von Figur 7 führen. Im Beispiel von Figur 8 ist im Gegensatz zum Beispiel von Figur 7 kein brennbares Zielgas in der Umgebung der Gasdetektionsvorrichtung 100 vorhanden, weswegen kein Zielgas in die Detektorkammer 6 fließt, egal wie hoch die Modulator-Temperatur T_15 ist. Die Temperatur T_10 des Detektors 10 bleibt konstant auf einem Referenz-Wert T_ref_10, der nur von der Stromstärke 1.1 und der Spannung U_10 abhängt.

Die Auswerteeinheit 9 entscheidet automatisch, ob die Detektor-Temperatur T_10 signifikant oszilliert, so wie dies in Figur 7 gezeigt ist, oder ob sie, so wie in Figur 8 gezeigt, konstant bleibt.

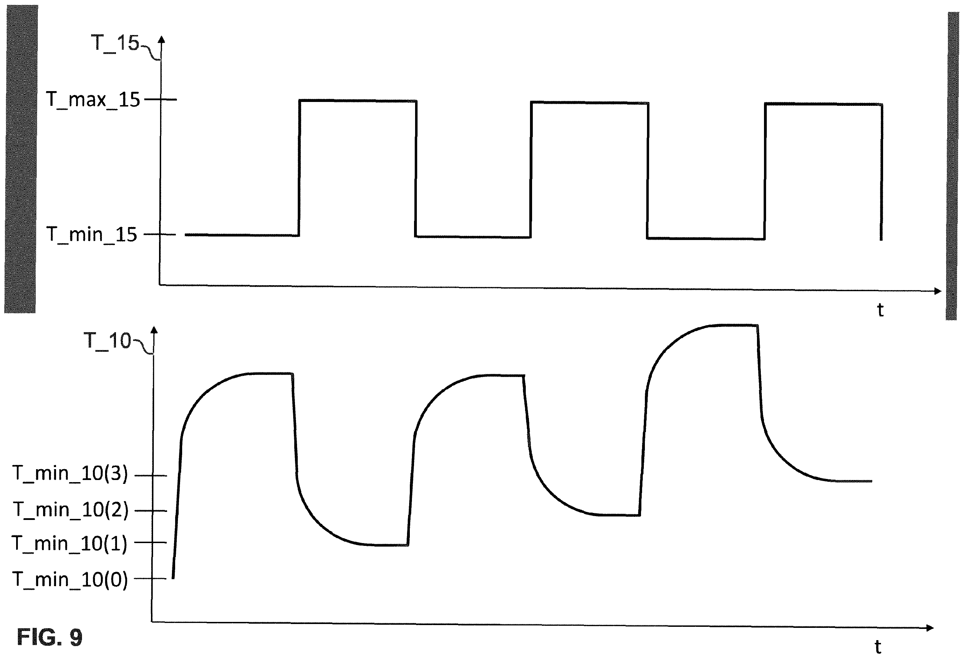

Die Darstellungen von Figur 7 und Figur 8 sind idealisierte Darstellungen. In der Praxis können sich mindestens eine Umgebungsbedingung oder eine Bedingung in der Detektorkammer 6 oder sowohl die Umgebungsbedingung als auch die Detektorkammer-Bedingung verändern. Figur 9 veranschaulicht schematisch die Auswirkung einer solchen Veränderung. Genauso wie in Figur 7 und Figur 8 ist auch in Figur 9 auf der x-Achse die Zeit t aufgetragen und auf der y-Achse die Modulator-Temperatur T_15 (oben) sowie die Detektor-Temperatur T_10 (unten). Aus der Umgebung fließt brennbares Zielgas durch die äußere Öffnung Ö1 hindurch in die Modulatorkammer 5 und dann, wenn die Modulator-Temperatur T_15 den niedrigeren Wert T_min_15 aufweist, weiter durch die innere Öffnung Ö2 in die Detektorkammer 6. Die Detektor-Temperatur T_10 oszilliert daher. Der Mindestwert T_min_10 verschiebt sich aber von Oszillation zu Oszillation, was durch die anwachsenden Mindestwerte T_min_10(0) < T_min_10(1) < T_min_10(2) < T_min_10(3) angedeutet ist. Auch in diesem Fall erkennt die Auswerteeinheit 9 die signifikante Oszillation der Detektor-Temperatur T_10 und entscheidet automatisch, dass ein brennbares Zielgas vorhanden ist.

Falls ein Zeitintervall, in dem die Modulator-Temperatur T_15 den höheren Wert T_max_15 annimmt, ausreichend lang ist, ist, so wird eine große Menge von brennbaren Zielgas in der Modulatorkammer 5 oxidiert. Daher ist am Ende dieses Zeitintervalls in der Modulatorkammer 5 nur eine relativ geringe Menge des Zielgases und daher in der Detektorkammer 6 nur eine vernachlässigbar kleine Menge des Zielgases vorhanden. Die Auswerteeinheit 9 verwendet die niedrige Detektor-Temperatur T_min_10(0) < T_min_10(1) < T_min_10(2) < T_min_10(3) am Ende dieses Zeitintervalls als die aktuellen Referenz-Temperatur des Detektors 10, also als denjenigen Wert der Detektor-Temperatur T_10, welche dann auftritt, wenn die Detektorkammer 6 frei von Zielgas ist. Diese Referenz-Temperatur lässt sich auch als ein Nullpunkt der Detektions-Größe des Detektors 10 bezeichnen. Der nachfolgende Anstieg der Detektor-Temperatur T_10 korreliert mit der aktuellen Konzentration des Zielgases in der Detektorkammer 6. Die erfindungsgemäße Gasdetektionsvorrichtung 100 führt daher eine automatische Justierung des Detektor-Nullpunkts durch. Möglich ist auch, eine Referenz-Spannung als einen Nullpunkt der Detektions-Größe zu ermitteln.

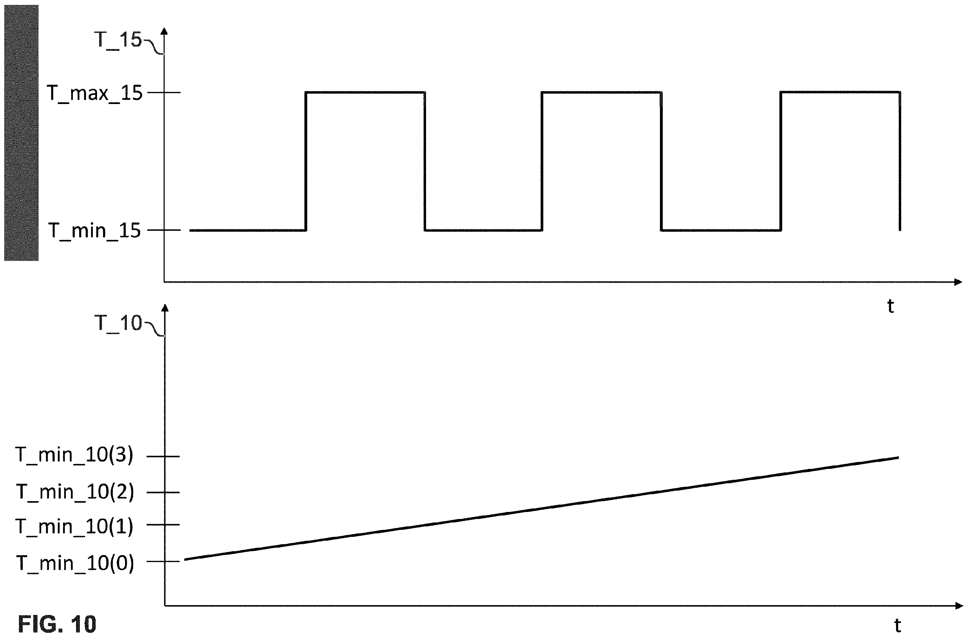

Figur 10 zeigt schematisch die beiden Verläufe, wobei die zeitlichen Verläufe der am Detektor zehn bzw. am Modulator 15 anliegenden elektrischen Spannungen sowie die Umgebungsbedingungen die gleichen sind wie in Figur 9, wobei aber kein Zielgas vorhanden ist. Aufgrund der sich verändernden Umgebungsbedingungen steigt die Detektor-Temperatur T_10 langsam an, ohne zu oszillieren.

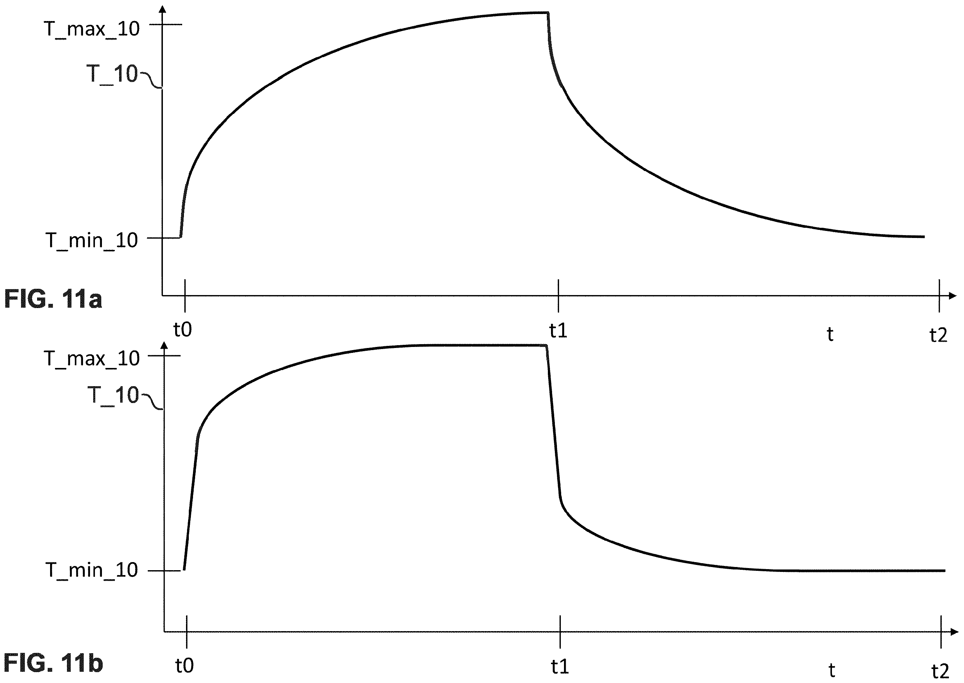

In den Darstellungen von Figur 2 bis Figur 10 wurde mindestens detektiert, ob ein brennbares Zielgas vorhanden ist oder nicht. Optional wurde durch Auswertung von Messwerten wenigstens näherungsweise die Konzentration des Zielgases ermittelt. In manchen Situationen ist es zusätzlich möglich, durch Auswertung des zeitlichen Verlauf der Detektor-Temperatur T_10 unterschiedliche brennbare Zielgase zu identifizieren. Dies veranschaulicht Figur 11 in einer idealisierten Darstellung.

Umgebungsluft mit einem brennbares Zielgas fließt aus dem Bereich B in die Modulatorkammer 5. Im Zeitraum von t0 bis t1 nimmt die Modulator-Temperatur T_15 den niedrigeren Wert T_min_15 an, im Zeitpunkt von t1 bis t2 den höheren Wert T_max_15. Daher fließt - idealisiert - im Zeitraum von t0 bis t1 eine größere Menge des brennbaren Zielgases in die Detektorkammer 6, im Zeitpunkt von t1 bis t2 eine geringere Menge oder gar kein brennbares Zielgas.

In Figur 11a) fließt ein brennbares Zielgas einer ersten Art in die Detektorkammer 6 und führt zu einem relativ langsamen Anstieg der Detektor-Temperatur T_10 bis zum Maximalwert T_max_10. In Figur 11b) fließt ein brennbares Zielgas einer zweiten Art in die Detektorkammer 6 und führt zu einem relativ raschen Anstieg der Detektor-Temperatur T_10 bis zum Maximalwert T_max_10. Die Auswerteeinheit 9 vermag diese beiden Situationen voneinander zu unterscheiden und dadurch automatisch zu entscheiden, ob in dem überwachten Bereich B überhaupt kein brennbares Zielgas oberhalb der Nachweisgrenze, Zielgas der ersten Art oder Zielgas der zweiten Art vorhanden ist.

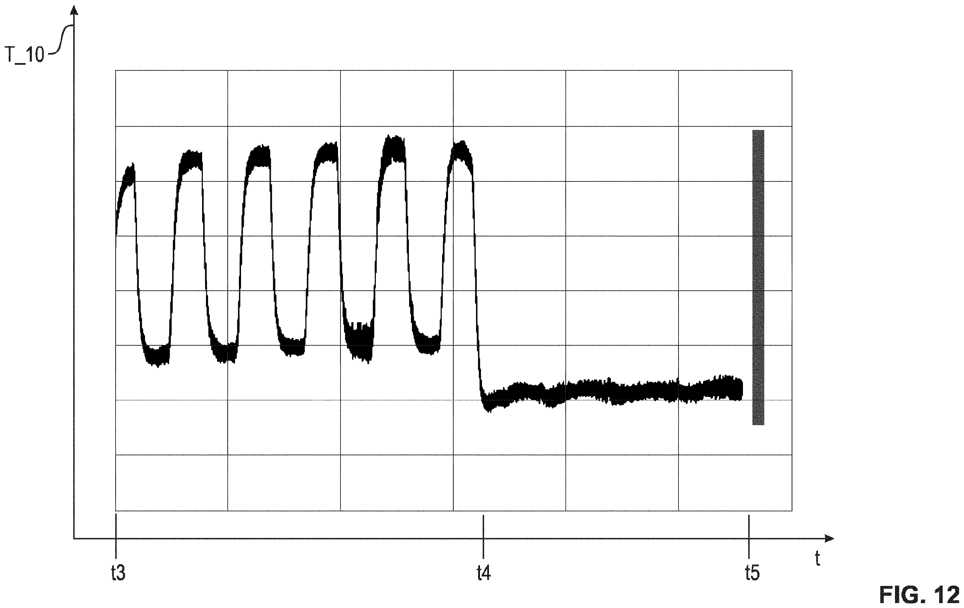

Figur 12 zeigt beispielhaft den zeitlichen Verlauf der Detektor-Temperatur T_10, der in einem realen Versuch gemessen wurde. Im gesamten Zeitraum von t3 bis t5 oszilliert die Modulator-Temperatur T_15, so wie dies in Figur 7 bis Figur 10 angedeutet ist. Im Zeitraum von t3 bis t4 befindet sich ein brennbares Zielgas in dem Bereich B, im Zeitraum von t4 bis t5 nicht. Wenn die Modulator-Temperatur T_15 den niedrigeren Wert T_min_15 aufweist, gelangt daher viel brennbares Zielgas in die Detektorkammer 6, ansonsten nur wenig brennbares Zielgas. Die Detektor-Temperatur T_10 oszilliert, und die Auswerteeinheit 9 detektiert im Zeitraum von t3 bis t4 dieses signifikante Oszillieren. Die Auswerteeinheit 9 detektiert im Zeitraum von t4 bis t5 hingegen kein signifikantes Oszillieren. Ein leichtes Oszillieren im gesamten Zeitraum von t3 bis t5 resultiert in diesem Fall zum einen daraus, dass die Modulatorkammer 5 und die Detektorkammer 6 thermisch nicht vollständig voneinander isoliert sind, und zum anderen aus variierenden Umgebungsbedingungen, veränderlichen Bedingungen in der Detektorkammer 6 und / oder einem unvermeidlichen Messrauschen. Die Auswerteeinheit 9 vermag dieses leichte Oszillieren automatisch von dem signifikanten Oszillieren im Zeitraum von t3 bis t4 zu unterscheiden und dadurch festzustellen, dass sich nur im Zeitraum von t3 bis t4 brennbares Zielgas in der Umgebung der Gasdetektionsvorrichtung 100 befindet.

**Bezugszeichenliste**

| | |
|---|---|
| 1 | Detektorkammer mit den Öffnungen Ö1 und Ö2, umschließt den Detektor 10, gehört zur Gasdetektionsvorrichtung 101 |
| 2 | Kompensatorkammer mit der Öffnung Ö2, umschließt den Kompensator 11, gehört zur Gasdetektionsvorrichtung 101 |
| 3 | elektrische Leitung, welche den Detektor 10 und den Kompensator 11 mit einer Spannungsquelle verbindet (Reihenschaltung) , gehört zur Gasdetektionsvorrichtung 101 |
| 3.1 | elektrische Leitung, welche den Detektor 10 mit einer Spannungsquelle 43 verbindet, gehört zur erfindungsgemäßen Gasdetektionsvorrichtung 100 |
| 3.2 | elektrische Leitung, welche den Modulator 15 mit einer Spannungsquelle 44 verbindet, gehört zur erfindungsgemäßen Gasdetektionsvorrichtung 100 |
| 4 | Explosionsschutzgitter in der äußeren Öffnung Ö1 |
| 5 | Modulatorkammer mit den Öffnungen Ö1 und Ö2, umschließt den Modulator 15 |
| 6 | Detektorkammer mit der inneren Öffnung Ö2, umschließt den Detektor 10 gasdicht |
| 7 | ansteuerbarer Spannungsregler in der elektrischen Leitung 3.2, beispielsweise ein Schalter, um eine Stromkreislauf zu schließen und zu öffnen |
| 9 | Auswerteeinheit, empfängt Messwerte von den Spannungssensoren 20.1 und 20.2 bzw. 20.1 und 20.3 |
| 10 | Detektor in Form eines Pellistors, in der Detektorkammer 1 bzw. 6, von der Spannungsquelle 43 über die elektrische Leitung 3 bzw. 3.1 mit Strom versorgt, gehört zur erfindungsgemäßen |
| | Gasdetektionsvorrichtung 100 und zur Gasdetektionsvorrichtung 101 |
| 11 | Kompensator in Form eines Pellistors in der Kompensatorkammer 2, über die elektrische Leitung 3 mit Strom versorgt, gehört zur Gasdetektionsvorrichtung 101 |
| 15 | Modulator in Form eines Pellistors in der Modulatorkammer 5, von der Spannungsquelle 44 über die elektrische Leitung 3.2 mit Strom versorgt, gehört zur erfindungsgemäßen Gasdetektionsvorrichtung 100 |
| 18 | thermische Barriere zwischen der Modulatorkammer 5 und der Detektorkammer 6 |
| 20.1 | Spannungs-Sensor, misst die am Detektor 10 anliegende elektrische Spannung U_10 |
| 20.2 | Spannungs-Sensor, misst die am Modulator 15 anliegende elektrische Spannung U_11 |
| 21.1 | Stromstärken-Sensor, misst die Stromstärke I.1 des durch den Detektor 10 fließenden Stroms |
| 21.2 | Stromstärken-Sensor, misst die Stromstärke I.2 des durch den Modulator 15 fließenden Stroms |
| 23 | heizendes Segment des Detektors 10, von der Keramikummantelung 25 umgeben |
| 24 | elektrische Verbindungen und mechanische Halterungen für das heizende Segment 23 |
| 25 | elektrisch isolierende Keramikummantelung um das heizende Segment 23 |
| 26 | Beschichtung auf und / oder in der Keramikummantelung 25, besteht aus einem katalytischen Material |
| 27 | Montageplatte des Detektors 10 |
| 30.1 | Temperatur-Sensor, misst die Temperatur T_10 des Detektors 10 |
| 30.2 | Temperatur-Sensor, misst die Temperatur T_15 des Modulators 15 |
| 40 | datenverarbeitendes Steuergerät, empfängt Messwerte von den Sensoren 20.1, 20.2, 21.1, 21.2, 30.1, 30.2, steuert die Spannungsveränderungseinheiten 41 und 42 an |
| 41 | Spannungsveränderungseinheit, welche die am Detektor 10 anliegende Spannung U_10 zu verändern vermag |
| 42 | Spannungsveränderungseinheit, welche die am Modulator 15 anliegende Spannung U_15 zu verändern vermag |
| 43 | Spannungsquelle für den Detektor 10 |
| 44 | Spannungsquelle für den Modulator 15 |
| 100 | erfindungsgemäße Gasdetektionsvorrichtung des Ausführungsbeispiels, umfasst den Detektor 10, den Modulator 15, die Detektorkammer 6, die Modulatorkammer 5, die Spannungs-Sensoren 20.1 und 20.2, die Stromstärken-Sensoren 21.1 und 21.2, das Steuergerät 40 mit der Auswerteeinheit 9, die Spannungsveränderungseinheiten 41 und 42 und die elektrischen Leitungen 3.1 und 3.2 |
| 101 | Gasdetektionsvorrichtung nach dem Stand der Technik, umfasst den Detektor 10, den Kompensator 11, die Detektorkammer 1, die Kompensatorkammer 2, die Auswerteeinheit 9 und die elektrische Leitung 3 |
| B | auf Zielgas zu überwachender Bereich |
| ΔI.1 | Regelabweichung zwischen I.1_soll und I.1 |
| ΔT_15 | Regelabweichung zwischen T_15 _ soll und T_15 |
| I.1 | Stromstärke in der elektrischen Leitung 3.1, zugleich Stärke des Stroms, der durch den Detektor 10 fließt, von dem Stromstärken-Sensor 21.1 gemessen |
| I.1_soll | vorgegebener zeitlicher Verlauf der gewünschten oder geforderten Stromstärke I.1 |
| I.2 | Stromstärke in der elektrischen Leitung 3.2, zugleich Stärke des Stroms, der durch den Detektor 10 fließt, von dem Stromstärken-Sensor 21.2 gemessen |
| I.3 | Stromstärke in der elektrischen Leitung 3, zugleich Stärke des Stroms, der durch den Detektor 10 und den Kompensator 11 fließt |
| Ö1 | Öffnung in der Detektorkammer 1 (Gasdetektionsvorrichtung 101) und in der Modulatorkammer 5 (erfindungsgemäße Gasdetektionsvorrichtung 100), nimmt das Explosionsschutzgitter 4 auf, fungiert als äußere Fluidverbindung |
| Ö2 | Öffnung zwischen der Detektorkammer 1 und der Kompensatorkammer 2 (Gasdetektionsvorrichtung 101) sowie Öffnung zwischen der Modulatorkammer 5 und der Detektorkammer 6 (erfindungsgemäße Gasdetektionsvorrichtung 100), fungiert als innere Fluidverbindung |
| T_10 | Temperatur des Detektors 10, korreliert mit der Konzentration des Zielgases in der Detektorkammer 6, in einer Ausgestaltung direkt von dem Temperatur-Sensor 30.1 gemessen |
| T_15 | Temperatur des Modulators 15, in einer Ausgestaltung direkt von dem Temperatur-Sensor 30.2 gemessen, von dem Steuergerät 40 geregelt |
| T_15 _ soll | vorgegebener zeitlicher Verlauf der gewünschten oder geforderten Modulator-Temperatur T_15 |
| T_max_10 | maximaler Wert der oszillierenden Detektor-Temperatur T_10 bei Vorhandensein eines brennbaren Zielgases |
| T_min_10 | minimaler Wert der oszillierenden Detektor-Temperatur T_10 bei Vorhandensein eines brennbaren Zielgases |
| T_ref_10 | Referenz-Wert der Detektor-Temperatur T_10, falls kein brennbares Zielgas vorhanden ist |
| T_max_15 | größerer Wert der Modulator-Temperatur T_15 |
| T_min_15 | kleinerer Wert der Modulator-Temperatur T_15 |
| U_10 | elektrische Spannung, die am Detektor 10 anliegt, vom Spannungs-Sensor 20.1 gemessen, in einer Ausgestaltung die Detektions-Größe |
| U_11 | elektrische Spannung, die am Kompensator 11 anliegt |
| U_15 | elektrische Spannung, die am Modulator 15 anliegt, vom Spannungs-Sensor 20.2 gemessen |

## Patentansprüche

1. Gasdetektionsvorrichtung (100) zum Überwachen eines Bereichs (B) auf das Vorhandensein eines brennbaren Zielgases (CH₄),
wobei die Gasdetektionsvorrichtung (100)
- einen Detektor (10),
- einen Modulator (15),
- eine Detektorkammer (6),
- eine Modulatorkammer (5),
- mindestens einen Detektor-Sensor (20.1, 21.1, 30.1) und
- eine signalverarbeitende Auswerteeinheit (9)
umfasst,
wobei die Modulatorkammer (5)
- den Modulator (15) umgibt und
- in einer äußeren Fluidverbindung (Ö1) mit dem zu überwachenden Bereich (B) steht,
wobei zwischen der Modulatorkammer (5) und der Detektorkammer (6) eine innere Fluidverbindung (Ö2) besteht,
wobei die Detektorkammer (6) den Detektor (10) - bis auf die innere Fluidverbindung (Ö2) - dergestalt von dem zu überwachenden Bereich (B) trennt, dass die Querschnittsfläche einer Fluidverbindung zwischen der Detektorkammer (6) und dem zu überwachenden Bereich (B) höchstens 25 % der Querschnittsfläche der inneren Fluidverbindung (Ö2) beträgt, bevorzugt höchstens 10 %,
wobei besonders bevorzugt die Detektorkammer (6) den Detektor (10) - bis auf die innere Fluidverbindung (Ö2) - von dem zu überwachenden Bereich (B) gasdicht trennt,
wobei die Gasdetektionsvorrichtung (100) dazu ausgestaltet ist, sowohl an den Modulator (15) als auch an den Detektor (10) eine elektrische Spannung (U_10, U_15) anzulegen,
wobei sowohl der Detektor (10) als auch der Modulator (15) so ausgestaltet sind, dass das Anlegen einer elektrischen Spannung (U_10, U_15) an den Detektor (10) bzw. den Modulator (15) bewirkt, dass ein Bauteil (23) des Detektors (10) bzw. ein Bauteil des Modulators (15) sich abhängig von der anliegenden elektrischen Spannung (U_10, U_15) erhitzt,
wobei ein Erhitzen des Detektors (10) bewirkt, dass ein zu detektierendes brennbares Zielgas (CH₄), welches sich in der Detektorkammer (6) befindet, abhängig von der Temperatur (T_10) des Detektors (10) oxidiert wird,
wobei ein Erhitzen des Modulators (15) bewirkt, dass ein zu detektierendes brennbares Zielgas (CH₄), welches sich in der Modulatorkammer (5) befindet, abhängig von der Temperatur (T_15) des Modulators (15) oxidiert wird,
wobei der Detektor (10) so ausgestaltet ist, dass ein Oxidieren eines Zielgases in der Detektorkammer (6) die Temperatur (T_10) des Detektors (10) vergrößert,
wobei der oder ein Detektor-Sensor (20.1, 21.1, 30.1) eine Detektions-Größe (T_10, U_10, 1.1) des Detektors (10) zu messen vermag und
wobei die Detektions-Größe (T_10, U_10, 1.1) die Detektor-Temperatur (T_10) oder ein mit der Detektor-Temperatur (T_10) korrelierender Parameter (U_10, 1.1) des Detektors (10) ist,
**dadurch gekennzeichnet, dass**
die Gasdetektionsvorrichtung (100) dazu ausgestaltet ist,
- an den Modulator (15) dergestalt eine elektrische Spannung (U_15) anzulegen, dass die Temperatur (T_15) des Modulators (15) zeitlich oszilliert,
insbesondere einen rechteckigen zeitlichen Verlauf aufweist, und
- an den Detektor (10) dergestalt eine elektrische Spannung (U_10) anzulegen, dass in einem vorgegebenen Auswerte-Zeitraum die durch das Anlegen der elektrischen Spannung (U_10) vergrößerte Temperatur (T_10) des Detektors (10) überhaupt nicht oder mit einer geringeren Amplitude als die Temperatur (T_15) des Modulators (15) zeitlich oszilliert,
wobei die Auswerteeinheit (9) dazu ausgestaltet ist,
- automatisch zu prüfen, ob die Detektions-Größe (T_10, U_10, 1.1) im Auswerte-Zeitraum synchron zum Oszillieren der Modulator-Temperatur (T_15) oszilliert oder nicht, und
- bei einer Detektion einer synchronen zeitlichen Oszillation der Detektions-Größe (T_10, U_10, 1.1) automatisch zu entscheiden, dass ein brennbares Zielgas (CH₄) in dem Bereich (B) vorhanden ist.

2. Gasdetektionsvorrichtung (100) nach Anspruch 1,
**dadurch gekennzeichnet, dass**
die Gasdetektionsvorrichtung (100) dazu ausgestaltet ist, an den Modulator (15) dergestalt eine elektrische Spannung (U_15) anzulegen,
dass die Modulator-Temperatur (T_15) in wenigstens fünf verschiedenen Zunahme-Zeiträumen zunimmt und in wenigstens fünf Abnahme-Zeiträumen abnimmt,
wobei jeder Abnahme-Zeitraum zeitlich auf jeweils einen Zunahme-Zeitraum folgt,
bevorzugt die Spannung (U_15) dergestalt anzulegen, dass die Modulator-Temperatur (T_15) in wenigstens zehn verschiedenen Zunahme-Zeiträumen zunimmt und in wenigstens zehn Abnahme-Zeiträumen abnimmt.

3. Gasdetektionsvorrichtung (100) nach einem der vorhergehenden Ansprüche,
**dadurch gekennzeichnet, dass**
die Gasdetektionsvorrichtung (100) mindestens einen Modulator-Sensor (20.2, 21.2, 30.2) umfasst,
wobei der oder ein Modulator-Sensor (20.2, 21.2, 30.2) dazu ausgestaltet ist, die Temperatur (T_15) des Modulators (15) oder einen mit der Modulator-Temperatur (T_15) korrelierenden Parameter (U_15, I.2) des Modulators (15) zu messen, und
wobei die Auswerteeinheit (9) dazu ausgestaltet ist, über das Vorhandensein oder Nicht-Vorhandensein eines brennbaren Zielgases (CH₄) abhängig vom Vorliegen oder Nichtvorliegen eines synchronen Oszillierens der Detektions-Größe (T_10, U_10, 1.1) und zusätzlich abhängig von der gemessenen Modulator-Temperatur (T_15) zu entscheiden.

4. Gasdetektionsvorrichtung (100) nach einem der vorhergehenden Ansprüche,
**dadurch gekennzeichnet, dass**
die Gasdetektionsvorrichtung (100) einen Modulator-Regelgrößen-Sensor (20.2, 21.2, 30.2) und ein signalverarbeitendes Steuergerät (40) umfasst,
wobei der Modulator-Regelgrößen-Sensor (20.2, 21.2, 30.2) dazu ausgestaltet ist, ein Maß für eine regelbare Größe (U_15, I.2, T_15) des Modulators (15) zu messen,
wobei die regelbare Größe (U_15, I.2, T_15) die Temperatur (T_15) des Modulators (15) ist oder mit der Temperatur (T_15) des Modulators (15) korreliert und
wobei das Steuergerät (40) dazu ausgestaltet ist, automatisch
unter Verwendung von Messwerten des Modulator-Regelgrößen-Sensors (20.2, 21.2, 30.2) die regelbare Größe (U_15, I.2, T_15) des Modulators (15) mit dem Regelungsziel zu regeln,
dass der tatsächliche zeitliche Verlauf dieser regelbaren Größe (U_15, I.2, T_15) einem vorgegebenen oszillierenden zeitlichen Verlauf (T_15_soll) der regelbaren Größe (T_15) folgt,
insbesondere einem rechteckigen zeitlichen Verlauf,
wobei die regelbare Größe (U_15, I.2, T_15) insbesondere
- die am Modulator (15) anliegende elektrische Spannung (U_15),
- die Stromstärke (I.2) des durch den Modulator (15) fließenden elektrischen Stroms,
- der elektrische Widerstand des Modulators (15),
- die vom Modulator (15) aufgenommene elektrische Leistung oder
- die Temperatur (T_15) des Modulators (15)
ist.

5. Gasdetektionsvorrichtung (100) nach einem der vorhergehenden Ansprüche,
**dadurch gekennzeichnet, dass**
die Gasdetektionsvorrichtung (100) einen Detektor-Regelgrößen-Sensor (20.1, 21.1. 30.1) und ein signalverarbeitendes Steuergerät (40) umfasst,
wobei der Detektor-Regelgrößen-Sensor (20.1, 21.1, 30.1) dazu ausgestaltet ist, ein Maß für eine regelbare Größe (1.1, U_10, T_10) des Detektors (10) zu messen,
wobei die regelbare Größe (1.1, U_10, T_10) des Detektors (10)
- die Temperatur (T_10) des Detektors (10) ist oder mit der Temperatur (T_15) des Detektors (10) korreliert und
- von der Detektions-Größe (T_10, U_10, 1.1) des Detektors (10) verschieden ist, und
wobei das Steuergerät (40) dazu ausgestaltet ist, automatisch
unter Verwendung von Messwerten des Detektor-Regelgrößen-Sensors (20.1, 21.1, 30.1) die am Detektor (10) anliegende elektrische Spannung (U_10) mit dem Regelungsziel zu regeln,
dass der tatsächliche zeitliche Verlauf der regelbare Größe (1.1, U_10, T_10) des Detektors (10) einem vorgegebenen zeitlichen Verlauf (1.1_soll) der regelbaren Größe (1.1, U_10, T_10) folgt.

6. Gasdetektionsvorrichtung (100) nach Anspruch 5,
**dadurch gekennzeichnet, dass**
die regelbare Größe (U_15, I.2, T_15) des Detektors (10)
- dann, wenn die Detektions-Größe des Detektors (10) nicht die elektrische Spannung ist, die am Detektor (10) anliegende elektrische Spannung (U_10) oder
- dann, wenn die Detektions-Größe des Detektors (10) nicht die Stromstärke ist, die Stromstärke (1.1) des durch den Detektor (10) fließenden Stroms oder
- dann, wenn die Detektions-Größe des Detektors (10) nicht der elektrische Widerstand ist, der elektrische Widerstand des Detektors (10) oder
- dann, wenn die Detektions-Größe des Detektors (10) nicht die elektrische Leistung ist, die vom Detektor (10) aufgenommene elektrische Leistung oder
- dann, wenn die Detektions-Größe des Detektors (10) nicht die Temperatur ist, die Temperatur (T_10) des Detektors (10)
ist.

7. Gasdetektionsvorrichtung (100) nach Anspruch 5 oder Anspruch 6,
**dadurch gekennzeichnet, dass**
das Regelungsziel ist, dass die regelbare Größe (1.1, U_10, T_10) des Detektors (10) im Auswerte-Zeitraum konstant bleibt.

8. Gasdetektionsvorrichtung (100) nach einem der vorhergehenden Ansprüche,
**dadurch gekennzeichnet, dass**
die Gasdetektionsvorrichtung (100) dazu ausgestaltet ist,
an den Modulator (15) dergestalt eine elektrische Spannung (U_15) anzulegen, dass die Temperatur (T_15) des Modulators (15) im Auswerte-Zeitraum zwischen einem minimalen Temperatur-Wert (T_min_15) und einen maximalen Temperatur-Wert (T_max_15) oszilliert, und
die Auswerteeinheit (9) dazu ausgestaltet ist,
- mindestens einmal einen Referenz-Messwert [T_min_10, T_min_10(0), T_min_10(1), T_min_10(2), T_min_10(3)] der Detektions-Größe (T_10, U_10, 1.1) zu ermitteln und
- den oder einen ermittelten Referenz-Messwert [T_min_10, T_min_10(0), T_min_10(1), T_min_10(2), T_min_10(3)] als einen aktuellen Referenz-Wert für die Detektions-Größe (T_10, U_10, 1.1) beim Nicht-Vorhandensein eines brennbaren Zielgases (CH₄) zu verwenden,
wobei die Auswerteeinheit (9) dazu ausgestaltet ist, als den Referenz-Messwert [T_min_10, T_min_10(0), T_min_10(1), T_min_10(2), T_min_10(3)] einen Wert zu ermitteln, den der oder ein Detektor-Sensor (20.1, 21.1, 30.1) im Auswerte-Zeitraum dann gemessen hat, wenn die Modulator-Temperatur (T_10) den maximalen Temperatur-Wert (T_max_15) annimmt,

9. Gasdetektionsvorrichtung (100) nach Anspruch 8,
**dadurch gekennzeichnet, dass**
die Auswerteeinheit (9) dazu ausgestaltet ist, eine Information über die aktuelle Konzentration und / oder die Art eines brennbaren Zielgases (CH₄) abhängig von dem Referenz-Messwert [T_min_10, T_min_10(0), T_min_10(1), T_min_10(2), T_min_10(3)] und einem nachfolgenden Anstieg der Detektions-Größe (T_10, U_10, 1.1) zu ermitteln.

10. Verfahren zum automatischen Überwachen eines Bereichs (B) auf das Vorhandensein eines brennbaren Zielgases (CH₄)
unter Verwendung einer Gasdetektionsvorrichtung (100), welche
- einen Detektor (10),
- einen Modulator (15),
- eine Detektorkammer (6),
- eine Modulatorkammer (5) und
- mindestens einen Detektor-Sensor (20.1, 21.1, 30.1)
umfasst,
wobei die Modulatorkammer (5) den Modulator (15) umgibt und in einer äußeren Fluidverbindung (Ö1) mit dem zu überwachenden Bereich (B) steht,
wobei zwischen der Modulatorkammer (5) und der Detektorkammer (6) eine innere Fluidverbindung (Ö2) besteht,
wobei die Detektorkammer (6) den Detektor (10) - bis auf die innere Fluidverbindung (Ö2) - dergestalt von dem zu überwachenden Bereich (B) trennt, dass die Querschnittsfläche einer Fluidverbindung zwischen der Detektorkammer (6) und dem zu überwachenden Bereich (B) höchstens 25 % der Querschnittsfläche der inneren Fluidverbindung (Ö2) beträgt, bevorzugt höchstens 10 %,
wobei besonders bevorzugt die Detektorkammer (6) den Detektor (10) - bis auf die innere Fluidverbindung (Ö2) - von dem zu überwachenden Bereich (B) gasdicht trennt,
wobei das Verfahren die Schritte umfasst, dass die Gasdetektionsvorrichtung (100)
- an den Modulator (15) eine elektrische Spannung (U_15) anlegt und
- an den Detektor (10) eine elektrische Spannung (U_10) anlegt, und
wobei das Verfahren die weiteren Schritte umfasst, dass
- das Anlegen der elektrischen Spannung (U_15) an den Modulator (15) bewirkt, dass sich ein Bauteil des Modulators (15) abhängig von der anliegenden Spannung (U_15) erhitzt,
- ein Erhitzen des Modulators (15) bewirkt, dass ein zu detektierendes brennbares Zielgas (CH₄), welches sich in der Modulatorkammer (5) befindet, abhängig von der Temperatur (T_15) des Modulators (15) oxidiert wird,
- das Anlegen der elektrischen Spannung (U_10) an den Detektor (10) bewirkt,
dass ein Bauteil (23) des Detektors (10) sich abhängig von der anliegenden elektrischen Spannung (U_10) erhitzt,
- ein Erhitzen des Detektors (10) bewirkt, dass ein zu detektierendes brennbares Zielgas (CH₄), welches sich in der Detektorkammer (6) befindet, abhängig von der Temperatur (T_10) des Detektors (10) oxidiert wird,
- ein Oxidieren eines Zielgases (CH₄) in der Detektorkammer (6) bewirkt, dass die Temperatur des Detektors (10) vergrößert wird, und
- der oder ein Detektor-Sensor (20.1, 21.1, 30.1) eine Detektions-Größe (T_10, U_10, 1.1) des Detektors (10) misst,
wobei die Detektions-Größe die Detektor-Temperatur (T_10) oder ein mit der Detektor-Temperatur (T_10) korrelierender Parameter (U_10, 1.1) des Detektors (10) ist,
**dadurch gekennzeichnet, dass**
das Verfahren die weiteren Schritte umfasst, dass
- die elektrische Spannung (U_15) dergestalt an den Modulator (15) angelegt wird, dass die Temperatur (T_15) des Modulators (15) zeitlich oszilliert,
insbesondere einen rechteckigen zeitlichen Verlauf aufweist,
- in einem vorgegebenen Auswerte-Zeitraum die durch das Anlegen der elektrischen Spannung (U_10) vergrößerte Temperatur (T_10) des Detektors (10) überhaupt nicht oder mit einer geringeren Amplitude als die Temperatur (T_15) des Modulators (15) zeitlich oszilliert,
- automatisch geprüft wird, ob die Detektions-Größe (T_10, U_10, 1.1) des Detektors (10) im Auswerte-Zeitraum synchron zum Oszillieren der Modulator-Temperatur (T_15) oszilliert oder nicht, und
- dann, wenn eine synchrone zeitliche Oszillation der Detektions-Größe (T_10, U_10, 1.1) detektiert wird, automatisch entschieden wird, dass ein brennbares Zielgas (CH₄) in dem Bereich (B) vorhanden ist.

## Claims

1. Gas detection apparatus (100) for monitoring a region (B) for the presence of a combustible target gas (CH₄),
wherein the gas detection apparatus (100) comprises
- a detector (10),
- a modulator (15),
- a detector chamber (6),
- a modulator chamber (5),
- at least one detector sensor (20.1, 21.1, 30.1) and
- a signal-processing evaluation unit (9),
wherein the modulator chamber (5)
- surrounds the modulator (15) and
- has an external fluid connection (Ö1) to the region (B) to be monitored,
wherein there is an internal fluid connection (Ö2) between the modulator chamber (5) and the detector chamber (6),
wherein the detector chamber (6) - with the exception of the internal fluid connection (Ö2) - isolates the detector (10) from the region (B) to be monitored in such a way that the cross-sectional area of a fluid connection between the detector chamber (6) and the region (B) to be monitored is no more than 25% of the cross-sectional area of the internal fluid connection (Ö2), preferably no more than 10%,
wherein the detector chamber (6) - with the exception of the internal fluid connection (Ö2) - particularly preferably isolates the detector (10) from the region (B) to be monitored in a gas-tight manner,
wherein the gas detection apparatus (100) is configured to apply a voltage (U_10, U_15) both to the modulator (15) and to the detector (10),
wherein both the detector (10) and the modulator (15) are configured so that applying a voltage (U_10, U_15) to the detector (10) or the modulator (15) causes an element (23) of the detector (10) or an element of the modulator (15) to heat on the basis of the applied voltage (U_10, U_15),
wherein heating of the detector (10) causes a combustible target gas (CH₄) that is to be detected and is in the detector chamber (6) to be oxidized on the basis of the temperature (T_10) of the detector (10),
wherein heating of the modulator (15) causes a combustible target gas (CH₄) that is to be detected and is in the modulator chamber (5) to be oxidized on the basis of the temperature (T_15) of the modulator (15),
wherein the detector (10) is configured such that oxidization of a target gas in the detector chamber (6) raises the temperature (T_10) of the detector (10),
wherein the or a detector sensor (20.1, 21.1, 30.1) is able to measure a detection variable (T_10, U_10, 1.1) of the detector (10) and
wherein the detection variable (T_10, U_10, I. 1) is the detector temperature (T_10) or a parameter (U_10, 1.1) of the detector (10) that correlates with the detector temperature (T_10),
**characterized in that**
the gas detection apparatus (100) is configured
- to apply a voltage (U_15) to the modulator (15) in such a way that the temperature (T_15) of the modulator (15) oscillates over time,
in particular has a square-wave time characteristic, and
- to apply a voltage (U_10) to the detector (10) in such a way that, in a predefined evaluation period, the raised temperature (T_10) of the detector (10) as a result of the voltage (U_10) being applied does not oscillate over time at all or oscillates over time with a smaller amplitude than the temperature (T_15) of the modulator (15),
wherein the evaluation unit (9) is configured
- to automatically check whether or not the detection variable (T_10, U_10, 1.1) oscillates in sync with the oscillation of the modulator temperature (T_15) in the evaluation period, and
- to respond to detection of a synchronous oscillation of the detection variable (T_10, U_10, 1.1) over time by automatically deciding that there is a combustible target gas (CH₄) in the region (B).

2. Gas detection apparatus (100) according to Claim 1,
**characterized in that**
the gas detection apparatus (100) is configured to apply a voltage (U_15) to the modulator (15) in such a way
that the modulator temperature (T_15) increases in at least five different increase periods and decreases in at least five decrease periods,
wherein each decrease period follows a respective increase period in time, preferably to apply the voltage (U_15) in such a way that the modulator temperature (T_15) increases in at least ten different increase periods and decreases in at least ten decrease periods.

3. Gas detection apparatus (100) according to either of the preceding claims,
**characterized in that**
the gas detection apparatus (100) comprises at least one modulator sensor (20.2, 21.2, 30.2),
wherein the or a modulator sensor (20.2, 21.2, 30.2) is configured to measure the temperature (T_15) of the modulator (15) or a parameter (U_15, I.2) of the modulator (15) that correlates with the modulator temperature (T_15), and
wherein the evaluation unit (9) is configured to decide about the presence or absence of a combustible target gas (CH₄) on the basis of the existence or nonexistence of a synchronous oscillation of the detection variable (T_10, U_10, 1.1) and additionally on the basis of the measured modulator temperature (T_15).

4. Gas detection apparatus (100) according to one of the preceding claims,
**characterized in that**
the gas detection apparatus (100) comprises a modulator controlled-variable sensor (20.2, 21.2, 30.2) and a signal-processing control unit (40),
wherein the modulator controlled-variable sensor (20.2, 21.2, 30.2) is configured to measure a level of a controllable variable (U_15, I.2, T_15) of the modulator (15),
wherein the controllable variable (U_15, I.2, T_15) is the temperature (T_15) of the modulator (15) or correlates with the temperature (T_15) of the modulator (15) and
wherein the control unit (40) is configured to automatically use measured values from the modulator controlled-variable sensor (20.2, 21.2, 30.2) to control the controllable variable (U_15, I.2, T_15) of the modulator (15) with the control aim,
**in that** the actual time characteristic of this controllable variable (U_15, I.2, T_15) follows a predefinable oscillating time characteristic (T_15_setpoint) of the controllable variable (T_15),
in particular a square-wave time characteristic,
wherein the controllable variable (U_15, I.2, T_15) is in particular
- the voltage (U_15) applied to the modulator (15),
- the amperage (1.2) of the electric current flowing through the modulator (15),
- the electrical resistance of the modulator (15),
- the electrical power drawn by the modulator (15)
- the temperature (T_15) of the modulator (15).

5. Gas detection apparatus (100) according to one of the preceding claims,
**characterized in that**
the gas detection apparatus (100) comprises a detector controlled-variable sensor (20.1, 21.1, 30.1) and a signal-processing control unit (40),
wherein the detector controlled-variable sensor (20.1, 21.1, 30.1) is configured to measure a level of a controllable variable (1.1, U_10, T_10) of the detector (10),
wherein the controllable variable (1.1, U_10, T_10) of the detector (10)
- is the temperature (T_10) of the detector (10) or correlates with the temperature (T_15) of the detector (10) and
- is different from the detection variable (T_10, U_10, 1.1) of the detector (10), and
wherein the control unit (40) is configured to automatically
use measured values from the detector controlled-variable sensor (20.1, 21.1, 30.1) to control the voltage (U_10) applied to the detector (10) with the control aim,
**in that** the actual time characteristic of the controllable variable (1.1, U_10, T_10) of the detector (10) follows a predefined time characteristic (1.1_setpoint) of the controllable variable (1.1, U_10, T_10).

6. Gas detection apparatus (100) according to Claim 5,
**characterized in that**
the controllable variable (U_15, I.2, T_15) of the detector (10),
- if the detection variable of the detector (10) is not the voltage, is the voltage (U_10) applied to the detector (10), or
- if the detection variable of the detector (10) is not the amperage, is the amperage (1.1) of the current flowing through the detector (10), or
- if the detection variable of the detector (10) is not the electrical resistance, is the electrical resistance of the detector (10), or
- if the detection variable of the detector (10) is not the electrical power, is the electrical power drawn by the detector (10), or
- if the detection variable of the detector (10) is not the temperature, is the temperature (T_10) of the detector (10).

7. Gas detection apparatus (100) according to Claim 5 or Claim 6,
**characterized in that**
the control aim is that the controllable variable (1.1, U_10, T_10) of the detector (10) remains constant in the evaluation period.

8. Gas detection apparatus (100) according to one of the preceding claims,
**characterized in that**
the gas detection apparatus (100) is configured
to apply a voltage (U_15) to the modulator (15) in such a way that the temperature (T_15) of the modulator (15) oscillates between a minimum temperature value (T_min_15) and a maximum temperature value (T_max_15) in the evaluation period, and
the evaluation unit (9) is configured
- to ascertain a reference measured value [T_min_10, T_min_10(0), T_min_10(1), T_min_10(2), T_min_10(3)] of the detection variable (T_10, U_10, 1.1) at least once and
- to use the or an ascertained reference measured value [T_min_10, T_min_10(0), T_min_10(1), T_min_10(2), T_min_10(3)] as a present reference value for the detection variable (T_10, U_10, 1.1) when a combustible target gas (CH₄) is absent,
wherein the evaluation unit (9) is configured to ascertain as the reference measured value [T_min_10, T_min_10(0), T_min_10(1), T_min_10(2), T_min_10(3)] a value that the or a detector sensor (20.1, 21.1, 30.1) has measured in the evaluation period when the modulator temperature (T_10) assumes the maximum temperature value (T_max_15).

9. Gas detection apparatus (100) according to Claim 8,
**characterized in that**
the evaluation unit (9) is configured to ascertain information about the present concentration and/or the type of a combustible target gas (CH₄) on the basis of the reference measured value [T_min_10, T_min_10(0), T_min_10(1), T_min_10(2), T_min_10(3)] and a subsequent rise in the detection variable (T_10, U_10, 1.1).

10. Method for automatically monitoring a region (B) for the presence of a combustible target gas (CH₄)
using a gas detection apparatus (100) that comprises
- a detector (10),
- a modulator (15),
- a detector chamber (6),
- a modulator chamber (5) and
- at least one detector sensor (20.1, 21.1, 30.1),
wherein the modulator chamber (5) surrounds the modulator (15) and has an external fluid connection (Ö1) to the region (B) to be monitored,
wherein there is an internal fluid connection (Ö2) between the modulator chamber (5) and the detector chamber (6),
wherein the detector chamber (6) - with the exception of the internal fluid connection (Ö2) - isolates the detector (10) from the region (B) to be monitored in such a way that the cross-sectional area of a fluid connection between the detector chamber (6) and the region (B) to be monitored is no more than 25% of the cross-sectional area of the internal fluid connection (Ö2), preferably no more than 10%,
wherein the detector chamber (6) - with the exception of the internal fluid connection (Ö2) - particularly preferably isolates the detector (10) from the region (B) to be monitored in a gas-tight manner,
wherein the method comprises the steps that the gas detection apparatus (100)
- applies a voltage (U_15) to the modulator (15) and
- applies a voltage (U_10) to the detector (10), and
wherein the method comprises the further steps that
- applying the voltage (U_15) to the modulator (15) causes an element of the modulator (15) to heat on the basis of the applied voltage (U_15),
- heating of the modulator (15) causes a combustible target gas (CH₄) that is to be detected and is in the modulator chamber (5) to be oxidized on the basis of the temperature (T_15) of the modulator (15),
- applying the voltage (U_10) to the detector (10) causes an element (23) of the detector (10) to heat on the basis of the applied voltage (U_10),
- heating of the detector (10) causes a combustible target gas (CH₄) that is to be detected and is in the detector chamber (6) to be oxidized on the basis of the temperature (T_10) of the detector (10),
- oxidization of a target gas (CH₄) in the detector chamber (6) causes the temperature of the detector (10) to be raised, and
- the or a detector sensor (20.1, 21.1, 30.1) measures a detection variable (T_10, U_10, 1.1) of the detector (10),
wherein the detection variable is the detector temperature (T_10) or a parameter (U_10, 1.1) of the detector (10) that correlates with the detector temperature (T_10),
**characterized in that**
the method comprises the further steps that
- the voltage (U_15) is applied to the modulator (15) in such a way that the temperature (T_15) of the modulator (15) oscillates over time,
in particular has a square-wave time characteristic,
- in a predefined evaluation period, the raised temperature (T_10) of the detector (10) as a result of the voltage (U_10) being applied does not oscillate over time at all or oscillates over time with a smaller amplitude than the temperature (T_15) of the modulator (15),
- an automatic check is performed to determine whether or not the detection variable (T_10, U_10, 1.1) of the detector (10) oscillates in sync with the oscillation of the modulator temperature (T_15) in the evaluation period, and
- if a synchronous oscillation of the detection variable (T_10, U_10, 1.1) over time is detected, it is automatically decided that there is a combustible target gas (CH₄) in the region (B).

## Revendications

1. Dispositif de détection de gaz (100) permettant de surveiller la présence d'un gaz combustible cible (CH₄) dans une région (B),
le dispositif de détection de gaz (100) comprenant
- un détecteur (10),
- un modulateur (15),
- un compartiment de détecteur (6),
- un compartiment de modulateur (5),
- au moins un capteur de détecteur (20.1, 21.1, 30.1) et
- une unité d'évaluation (9) traitant les signaux,
le compartiment de modulateur (5)
- entourant le modulateur (15) et
- se trouvant en liaison fluidique externe (Ö1) avec la région à surveiller (B), une liaison fluidique interne (Ö2) étant constituée entre le compartiment de modulateur (5) et le compartiment de détecteur (6),
le compartiment de détecteur (6) séparant le détecteur (10) - à l'exception de la liaison fluidique interne (Ö2) - et la région à surveiller (B) de telle manière que la surface en coupe transversale d'une liaison fluidique entre le compartiment de détecteur (6) et la région à surveiller (B) est inférieure ou égale à 25 %, de manière préférée inférieure ou égale à 10 %, de la surface en coupe transversale de la liaison fluidique interne (Ö2),
le compartiment de détecteur (6) séparant le détecteur (10) - à l'exception de la liaison fluidique (Ö2) - et la région à surveiller (B) de manière particulièrement préférée de manière étanche aux gaz,
le dispositif de détection de gaz (100) étant conçu pour appliquer une tension électrique (U_10, U_15) à la fois au modulateur (15) et au détecteur (10),
le détecteur (10) et le modulateur (15) étant conçus de sorte que l'application d'une tension électrique (U_10, U_15) au détecteur (10) ou au modulateur (15) provoque la mise en température d'un composant (23) du détecteur (10) ou d'un composant du modulateur (15) en fonction de la tension électrique (U_10, U_15) appliquée,
la mise en température du détecteur (10) provoquant, en fonction de la température (T_10) du détecteur (10), l'oxydation d'un gaz combustible cible (CH₄) à détecter se trouvant dans le compartiment de détecteur (6),
la mise en température du modulateur (15) provoquant, en fonction de la température (T_15) du modulateur (15), l'oxydation d'un gaz combustible cible (CH₄) à détecter se trouvant dans le compartiment de modulateur (5),
le détecteur (10) étant conçu de sorte que l'oxydation d'un gaz cible dans le compartiment de détecteur (6) augmente la température (T_10) du détecteur (10),
le ou un capteur de détecteur (20.1, 21.1, 30.1) étant capable de mesurer une variable de détection (T_10, U_10, 1.1) du détecteur (10) et
la variable de détection (T_10, U_10, 1.1) étant la température de détecteur (T_10) ou un paramètre (U_10, 1.1) du détecteur (10) en corrélation avec la température de détecteur (T_10),
**caractérisé en ce que**
le dispositif de détection de gaz (100) est conçu pour
- appliquer au modulateur (15) une tension électrique (U_15) de telle manière que la température (T_15) du modulateur (15) oscille dans le temps et
présente en particulier une courbe temporelle rectangulaire, et
- appliquer au détecteur (10) une tension électrique (U_10) de telle manière que la température (T_10) du détecteur (10) augmentée par l'application de la tension électrique (U_10) n'oscille pas du tout au cours d'une période d'évaluation prédéterminée, ou avec une amplitude inférieure à la température (T_15) du modulateur (15),
l'unité d'évaluation (9) étant conçue pour
- vérifier automatiquement si, pendant la période d'évaluation, la variable de détection (T_10, U_10, 1.1) oscille ou non de manière synchrone par rapport à l'oscillation de la température de modulateur (T_15), et
- en cas de détection d'une oscillation temporelle synchrone de la variable de détection (T_10, U_10, 1.1), décider automatiquement de la présence d'un gaz combustible cible (CH₄) dans la région (B).

2. Dispositif de détection de gaz (100) selon la revendication 1,
**caractérisé en ce que**
le dispositif de détection de gaz (100) est conçu
pour appliquer une tension électrique (U_15) au modulateur (15) de telle manière que la température de modulateur (T_15) augmente au cours d'au moins cinq périodes d'augmentation différentes et diminue au cours d'au moins cinq périodes de diminution,
chaque période de diminution succédant temporellement à une période d'augmentation respective,
de manière préférée pour appliquer la tension (U_15) de telle manière que la température de modulateur (T_15) augmente au cours d'au moins dix périodes d'augmentation différentes et diminue au cours d'au moins dix périodes de diminution.

3. Dispositif de détection de gaz (100) selon l'une quelconque des revendications précédentes,
**caractérisé en ce que**
le dispositif de détection de gaz (100) comprend au moins un capteur de modulateur (20.2, 21.2, 30.2),
le ou un capteur de modulateur (20.2, 21.2, 30.2) étant conçu pour mesurer la température (T_15) du modulateur (15) ou un paramètre (U_15, I.2) du modulateur (15) en corrélation avec la température de modulateur (T_15), et
l'unité d'évaluation (9) étant conçue pour décider de la présence ou de l'absence d'un gaz combustible cible (CH4) en fonction de la présence ou de l'absence d'une oscillation synchrone de la variable de détection (T_10, U_10, 1.1) et en outre en fonction de la température de modulateur (T_15) mesurée.

4. Dispositif de détection de gaz (100) selon l'une quelconque des revendications précédentes,
**caractérisé en ce que**
le dispositif de détection de gaz (100) comprend un capteur de variable de réglage de modulateur (20.2, 21.2, 30.2) et un appareil de commande (40) traitant les signaux,
le capteur de variable de réglage de modulateur (20.2, 21.2, 30.2) étant conçu pour réaliser une mesure d'une variable de réglage (U_15, I.2, T_15) du modulateur (15), la variable de réglage (U_15, I.2, T_15) étant la température (T_15) du modulateur (15) ou étant en corrélation avec la température (T_15) du modulateur (15) et
le dispositif de commande (40) étant conçu pour régler de manière automatique la variable de réglage (U_15, I.2, T_15) du modulateur (15) avec l'objectif de réglage en utilisant des valeurs de mesure du capteur de variable de réglage de modulateur (20.2, 21.2, 30.2),
la courbe temporelle effective de ladite variable de réglage (U_15, I.2, T_15) suit une courbe temporelle (T_15_soll) oscillante prédéterminée de la variable de réglage (T_15),
en particulier une courbe temporelle rectangulaire,
la variable de réglage (U_15, I.2, T_15) étant en particulier
- la tension électrique (U_15) appliquée au modulateur (15),
- l'intensité (I.2) du courant électrique traversant le modulateur (15),
- la résistance électrique du modulateur (15),
- la puissance électrique absorbée par le modulateur (15) ou
- la température (T_15) du modulateur (15).

5. Dispositif de détection de gaz (100) selon l'une quelconque des revendications précédentes,
**caractérisé en ce que**
- le dispositif de détection de gaz (100) comprend un capteur de variable de réglage de détecteur (20.1, 21.1. 30.1) et un appareil de commande (40) traitant les signaux, le capteur de variable de réglage de détecteur (20.1, 21.1, 30.1) étant conçu pour réaliser une mesure d'une variable de réglage (1.1, U_10, T_10) du détecteur (10),
la variable de réglage (1.1, U_10, T_10) du détecteur (10)
- étant la température (T_10) du détecteur (10) ou étant en corrélation avec la température (T_15) du détecteur (10) et
- étant différente de la variable de détection (T_10, U_10, 1.1) du détecteur (10), et
le dispositif de commande (40) étant conçu pour régler de manière automatique la tension électrique (U_10) appliquée au détecteur (10) avec l'objectif de réglage en utilisant des valeurs de mesure du capteur de variable de réglage de détecteur (20.1, 21.1, 30.1),
la courbe temporelle effective de la variable de réglage (1.1, U_10, T_10) du détecteur (10) suit une courbe temporelle (1.1_soll) prédéterminée de la variable de réglage (1.1, U_10, T_10).

6. Dispositif de détection de gaz (100) selon la revendication 5,
**caractérisé en ce que**
la variable de réglage (U_15, I.2, T_15) du détecteur (10)
- est donc la tension électrique (U_10) appliquée au détecteur (10), si la variable de détection du détecteur (10) n'est pas la tension électrique, ou
- est donc l'intensité de courant (1.1) du courant traversant le détecteur (10), si la variable de détection du détecteur (10) n'est pas l'intensité de courant, ou
- est donc la résistance électrique du détecteur (10), si la variable de détection du détecteur (10) n'est pas la résistance électrique, ou
- est donc la puissance électrique absorbée par le détecteur (10), si la variable de détection du détecteur (10) n'est pas la puissance électrique, ou
- est donc la température (T_10) du détecteur (10), si la variable de détection du détecteur (10) n'est pas la température,.

7. Dispositif de détection de gaz (100) selon la revendication 5 ou la revendication 6,
**caractérisé en ce que**
l'objectif de réglage est que la variable de réglage (1.1, U_10, T_10) du détecteur (10) reste constante pendant la période d'évaluation.

8. Appareil (100) selon l'une quelconque des revendications précédentes, **caractérisé en ce que**
le dispositif de détection de gaz (100) est conçu pour
appliquer une tension électrique (U_15) au modulateur (15) de telle manière que la température (T_15) du modulateur (15) oscille pendant la période d'évaluation entre une valeur de température minimale (T_min_15) et une valeur de température maximale (T_max_15), et
l'unité d'évaluation (9) est conçue pour
- déterminer au moins une fois une valeur de mesure de référence [T_min_10, T_min_10(0), T_min_10(1), T_min_10(2), T_min_10(3)] de la variable de détection (T_10, U_10, 1.1) et
- utiliser la ou une valeur de mesure de référence [T_min_10, T_min_10(0), T_min_10(1), T_min_10(2), T_min_10(3)] déterminée comme valeur de référence actuelle pour la variable de détection (T_10, U_10, 1.1) en l'absence d'un gaz combustible cible (CH4),
- l'unité d'évaluation (9) étant conçue pour déterminer, en tant que valeur de mesure de référence [T_min_10, T_min_10(0), T_min_10(1), T_min_10(2), T_min_10(3)], une valeur que le ou un capteur de détecteur (20.1, 21.1, 30.1) a mesurée pendant la période d'évaluation lorsque la température de modulateur (T_10) prend la valeur de température maximale (T_max_15).

9. Dispositif de détection de gaz (100) selon la revendication 8,
**caractérisé en ce que**
l'unité d'évaluation (9) est conçue pour déterminer une information sur la concentration actuelle et/ou le type de gaz combustible cible (CH₄) en fonction de la valeur de mesure de référence [T_min_10, T_min_10(0), T_min_10(1), T_min_10(2), T_min_10(3)] et d'une augmentation ultérieure de la variable de détection (T_10, U_10, I.1).

10. Procédé de surveillance automatique de la présence d'un gaz combustible cible (CH₄) dans une région (B)
en utilisant un dispositif de détection de gaz (100) comprenant
- un détecteur (10),
- un modulateur (15),
- un compartiment de détecteur (6),
- un compartiment de modulateur (5) et
- au moins un capteur de détecteur (20.1, 21.1, 30.1),
le compartiment de modulateur (5) entourant le modulateur (15) et étant en liaison fluidique externe (Ö1) avec la région à surveiller (B),
une liaison fluidique interne (Ö2) étant constituée entre le compartiment de modulateur (5) et le compartiment de détecteur (6),
le compartiment de détecteur (6) séparant le détecteur (10) - à l'exception de la liaison fluidique interne (Ö2) - et la région à surveiller (B) de telle manière que la surface en coupe transversale d'une liaison fluidique entre le compartiment de détecteur (6) et la région à surveiller (B) est inférieure ou égale à 25 %, de manière préférée est inférieure ou égale à 10 %, de la surface en coupe transversale de la liaison fluidique interne (Ö2*)*,
le compartiment de détecteur (6) sépare le détecteur (10) - à l'exception de la liaison fluidique interne (Ö2) - et la région à surveiller (B) de manière particulièrement préférée de manière étanche aux gaz,
le procédé comprenant les étapes ci-dessous consistant pour le dispositif de détection de gaz (100) à :
- appliquer une tension électrique (U_15) au modulateur (15) et
- appliquer une tension électrique (U_10) au détecteur (10), et
le procédé comprenant les étapes supplémentaires ci-dessous :
- l'application de la tension électrique (U_15) au modulateur (15) provoque la mise en température d'un composant du modulateur (15) en fonction de la tension (U_15) appliquée,
- la mise en température du modulateur (15) provoque, en fonction de la température (T_15) du modulateur (15), l'oxydation d'un gaz combustible cible (CH4) à détecter se trouvant dans le compartiment de modulateur (5),
- l'application de la tension électrique (U_10) au détecteur (10) provoque la mise en température d'un composant (23) du détecteur (10) en fonction de la tension électrique (U_10) appliquée,
- la mise en température du détecteur (10) provoque, en fonction de la température (T_10) du détecteur (10), l'oxydation d'un gaz combustible cible (CH4) à détecter se trouvant dans le compartiment de détecteur (6),
- l'oxydation d'un gaz cible (CH4) dans le compartiment de détecteur (6) provoque une augmentation de la température du détecteur (10), et
- le ou un capteur de détecteur (20.1, 21.1, 30.1) mesure une variable de détection (T_10, U_10, 1.1) du détecteur (10),
la variable de détection étant la température de détecteur (T_10) ou un paramètre (U_10, 1.1) du détecteur (10) en corrélation avec la température de détecteur (T_10),
**caractérisé en ce que**
le procédé comprend les étapes ci-dessous :
- la tension électrique (U_15) est appliquée au modulateur (15) de telle manière que la température (T_15) du modulateur (15) oscille dans le temps et présente en particulier une courbe temporelle rectangulaire,
- au cours d'une période d'évaluation prédéterminée, la température (T_10) du détecteur (10) augmentée par l'application de la tension électrique (U_10) n'oscille pas du tout ou avec une amplitude inférieure à la température (T_15) du modulateur (15),
- l'oscillation ou l'absence d'oscillation de la variable de détection (T_10, U_10, 1.1) du détecteur (10) de manière synchrone par rapport à l'oscillation de la température de modulateur (T_15) au cours de la période d'évaluation est vérifiée de manière automatique, et
- si une oscillation temporelle synchrone de la variable de détection (T_10, U_10, 1.1) est détectée, la présence d'un gaz combustible cible (CH4) dans la région (B) est décidé de manière automatique.
